# EUROPEAN PATENT APPLICATION

(11) **EP 4 227 324 A1**
(43) Date of publication of application: **16.08.2023**
(21) Application number: 22382121.6
(22) Date of filing: 15.02.2022
(51) Int. Cl.: C08B 37/08, A61K 31/722

(54) **ANTIVIRAL SULFATED CHITOSAN DERIVATIVES**

(71) Applicant: Consejo Superior De Investigaciones Científicas (CSIC), 28006 Madrid (ES); Universitat de Valéncia, 46010 Valencia (ES)
(72) Inventor: Fernández-Mayoralas Álvarez, Alfonso, 28006 Madrid (ES); Revuelta Crespo, Julia, 28006 Madrid (ES); García-Junceda Redondo, Eduardo, 28006 Madrid (ES); Bastida Codina, Agatha, 28006 Madrid (ES); Geller, Ron, 46010 Valencia (ES); Francés Gómez, Clara, 46010 Valencia (ES); Latorre Roselló, Víctor, 46010 Valencia (ES)
(74) Representative: Pons

(57) **Abstract**

The invention relates to sulfated chitosan derivatives of formula I, wherein the meanings for the various substituents are as disclosed in the description, for their use in the treatment and/or prevention of a viral infection, particularly wherein the viral infection is caused by SARS-CoV-2 such as COVID-19 or RSV.

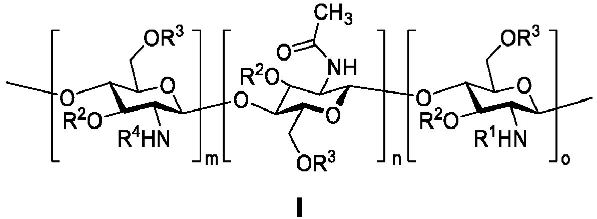

## Description

The invention relates to sulfated chitosan derivatives of formula I for their use in the treatment and/or prevention of viral infections, particularly wherein the viral infection is caused by SARS-CoV-2 or respiratory syncytial virus (RSV).

### BACKGROUND ART

In the field of viral infections, the recent emergence of Severe Acute Respiratory Syndrome Coronavirus (SARS-CoV-2) in Wuhan (China) in late 2019 and its subsequent spread to the rest of the world has created a pandemic situation unprecedented in modern history (Zhu, N. et al. N. Engl. J. Med. (2019) 382, 727; Wu, F. et al. Nature (2020) 579, 265; Perlman, S. N. Engl. J. Med. (2020) 382, 760; Gates, B. N. Engl. J. Med. (2020) 382, 1677). SARS-CoV-2 is an enveloped RNA beta-coronavirus, which has been identified as the causative pathogen of coronavirus disease 2019 (COVID-19). The spike glycoprotein in SARS-CoV-2 (SGP) interacts with the human angiotensin converting enzyme 2 (hACE2) receptor to gain entry into a cell to initiate infection. The bound virus is then endocytosed into the cell, where SGP is acted upon by the endosomal protease TMPRSS2 to allow envelope fusion and viral entry (Hoffmann, M. et al. Cell (2020) 181, 271). It is known that a range of pathogens, especially viruses, uses GAGs proteoglycans as co-receptors, allowing the virus to adhere to the surface of the cell, elevating the local concentration of viral particles and thereby increasing effective infection rates. There is evidence that SPG also interacts with GAGs in a sequence-dependent manner (Kim, S. Y. et al. Antiviral Res. (2020) 181, 104873; Liu, L. et al. ACS Cent. Sci. (2021) 7, 1009). GAGs are a family of linear sulfated polysaccharides found on the surface of virtually all mammalian cells, and commonly includes chondroitin sulfate (CS) and heparan sulfate (HS). Previous studies using isolated SARS-CoV-2 SGP monomer or trimer have shown that SARS-CoV-2 SGP has high affinity to heparin (Zhan, Q. et al. Cell Discovery (2020) 6, 80), a specialized member of the HS family that is highly sulfated and commonly used clinically as an anti-coagulant drug. It was also reported that heparin was capable of inhibiting infection of SARS-CoV-2 in Vero cell culture in a concentration-dependent fashion (Mycroft-West, C. J. et al. Thromb. Haemostasis (2020) 12, 1700). These results support a model of SARS-CoV-2 attachment and entry, where the virus employ HSs as adhesion molecules to increase density of the virus on the cell surface and facilitating the interaction between the protein S, the main mediator of viral entry, and its cellular receptor, the ACE2 (Mandel Clausen, T. et al. Cell (2020) 183, 1043). Thus, and according to this model, the virus adheres to HSs through the SGP of the viral envelope, in a stage prior to the union of the virus to its cellular receptor (Zhan, Q. et al. Cell Discovery (2020) 6, 80).

On the other hand, Respiratory Syncytial Virus (RSV) is an enveloped single-stranded negative-sense RNA virus belonging to the genus Pneumovirus of the family Paramyxoviridae (Esposito, S. et al. PLoS One (2015) 10, e0129369). It is the leading cause of bronchiolitis and pneumonia in infants and young children worldwide. More than half of all children are seropositive for RSV by 1 year of age, and almost all children have been infected by 2 years of age (Shay, D.K. et al. J. Infect. Dis. (2001) 183, 16). Moreover, RSV is a pathogen of considerable importance in immunocompromised adults and the elderly, particularly in those with chronic obstructive pulmonary disease (Falsey, A.R. et al. Am. J. Respir. Crit. Care Med. (2006), 173, 639). Currently, no vaccine for RSV is available and the management of RSV infection is primarily a matter of treating symptoms (Corsello, G. J. Chemother. (2007) 19, 12), and antiviral treatment is limited to the use of ribavirin, a drug which has controversial activity and is associated with significant side effects (Storey S. Nat. Rev. Drug Discov. (2010) 9, 15). Palivizumab, a humanized monoclonal antibody, has been approved for the immunoprophylaxis of RSV infection in just one narrowly defined patient group: high-risk prematurely born infants (Johnson, S. et al. J. Infect. Dis. (1997) 176, 1215). However, a major problem with palivizumab is its high cost, which may lead to the progressive restriction of its use (Steward, D.L. J. Manag. Care Pharm. (2010) 16, 509).

Numerous publications support that Respiratory Syncytial Virus (RSV) binding to cells involves an initial interaction between the viral envelope proteins G and F and HSs (Crim, R.L. et al. J. Virol. (2007) 81, 261; Feldman, S.A. et al. J. Virol. (2000) 74, 6442; Feldman, S.A. et al. J. Virol. (1999) 73, 6610). Subsequently, the virus engages secondary receptors: nucleolin (Tayyari, F. et al. Nat. Med. (2011) 17, 1132), ICAM1 (Behera, A.K. et al. Biochem. Biophys. Res. Commun. (2001) 280, 188), C-X3-C motif chemokine receptor 1 (Harcourt, J. J. Immunol. (2006) 176, 1600), and annexin II (Malhotra, R. et al. Microbes Infect. (2003) 5, 123). These interactions induce a conformation change of the F protein with exposure of its fusion peptide, promoting fusion of the viral envelope and the cell plasma membrane and release of the nucleocapside in the cytoplasm (Gilman, M.S. et al. PLoS Pathog. (2015) 11, e1005035). In addition, different competition experiments with heparin and other sulfated molecules, enzymatic removal for HSs from the cellular surface, as well as inhibition of sulfation have shown that RSV initial attachment to cells is HS-dependent (Hallak, L.K. et al. J. Virol. (2000) 74, 10508; Crim, R.L. et al. J. Virol. (2007) 81, 261; Feldman, S.A. et al. J. Virol. (2000) 74, 6442). Finally, highly sulfated polysaccharide derivatives have showed to be RSV inhibitors (Cagno, V. et al. Antimicrob. Agents Chemother. (2014) 58, 4782).

Finally, a consistent amount of data supports the natural dependence of other viruses on HS-proteoglycans for their attachment to the host cells such as Human metapneumovirus (Klimyte, E.M. et al. J. Virol. (2016) 90, 9237), Ebola Virus (EBOV) (Tamhankar, M. et al. Virol.J. (2018) 15, 135); Herpes Simplex Virus (HSV) (Shukla, D. et al. Cell (1999) 99, 13), Human papillomavirus (HPV) (Giroglou, T. et al. J. Virol. (2001) 75, 1565), Dengue Virus (DENV) (Cagno, V. et al. Pathogens (2021) 9, 935) and others (Cagno, V. et al. Viruses (2019) 11, 596).

Chitosan sulfates (ChitS) have demonstrated the ability to mimic HS function (Doncel-Perez, E. et al. Carbohydr. Polym (2018) 191, 225; Revuelta, J. et al. ACS Appl. Mater. Interfaces (2020) 12, 25534; Revuelta, J. et al. Mater. Horiz. (2021) 8, 2596). Accordingly, it would be desirable to provide new compounds that, acting as HS mimics, can inhibit the adhesion of the virus to HSs and, in consequence, block the entry of the virus to the cell. Such compounds could be useful for the treatment and/or prevention of viral infections and, particularly, for the treatment and/or prevention of infections caused by SARS-CoV-2, RSV, or other viruses.

### SUMMARY OF THE INVENTION

The present invention relates to the preparation of sulfated chitosan derivatives of formula I which are mimics of HSs, naturally occurring sulfated polysaccharides ubiquitously present in cells with important biological implications.

Accordingly, a first aspect of present invention relates to a compound of formula I: 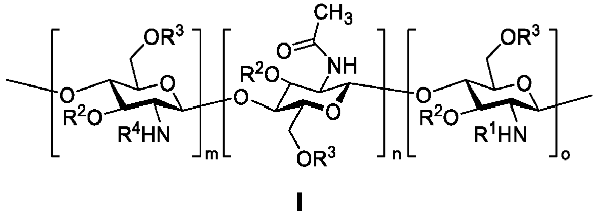 each of R¹, R² or R³ independently represents H or -SO₃M; with the proviso that total sulfation degree of compound expressed by Z is of between 0.05 and 3, and wherein Z is calculated in base to elemental analysis of compound according to this general formula: Z = (S% /32.06)/(N%/14.01); wherein S% and N% represent the percentage of each element by weight
M represents a monovalent cation, preferably pyridinium cation, an ammonium cation or an alkali metal cation;
R⁴ represents -CO-(CH₂)₇-CH=CH-(CH₂)₇-CH₃;
m represents from 0 to 1760 repeating units;
n represents from 1 to 1100 repeating units; and
o represents from 1 to 2100 repeating units.

In another embodiment the invention relates to the compound of formula I as defined above, wherein M is an ammonium cation selected from NH₄⁺, NMe₃H⁺, NEt₃H⁺ or NBU₃H⁺.

In another embodiment the invention relates to the compound of formula I as defined above, wherein M is an alkali metal cation selected from Na⁺ K⁺, Cs⁺ or Li⁺ and preferably selected from Na⁺.

In another embodiment the invention relates to the compound of formula I as defined above, wherein m is 0, R¹ is H and R² and/or R³ are H or -SO₃M.

In another embodiment the invention relates to the compound of formula I as defined above, wherein m is 0, R¹ is H or -SO₃M and R² and R³ are H.

In another embodiment the invention relates to the compound of formula I as defined above, wherein m represents from 0 to 200 repeating units, and preferably wherein m represents from 0 to 100 repeating units.

In another embodiment the invention relates to the compound of formula I as defined above, wherein m represents from 1 to 1760 repeating units, preferably wherein m represents from 1 to 200 repeating units, and more preferably wherein m represents from 1 to 100 repeating units.

In another embodiment the invention relates to the compound of formula I as defined above, wherein n represents from 1 to 500 repeating units, and preferably n represents from 1 to 200 repeating units.

In another embodiment the invention relates to the compound of formula I as defined above, wherein o represents from 1 to 700, and preferably wherein o represents from 1 to 400 repeating units.

In another embodiment the invention relates to the compound of formula I as defined above, wherein:
m represents from 0 to 200 repeating units, and preferably wherein m represents from 0 to 100 repeating units;
n represents from 1 to 500 repeating units, and preferably n represents from 1 to 200 repeating units; and
o represents from 1 to 700, and preferably wherein o represents from 1 to 400 repeating units.

In another embodiment the invention relates to the compound of formula I as defined above, wherein:
m represents from 1 to 1760 repeating units, preferably wherein m represents from 1 to 200 repeating units, and more preferably wherein m represents from 1 to 100 repeating units;
n represents from 1 to 500 repeating units, and preferably n represents from 1 to 200 repeating units; and
o represents from 1 to 700, and preferably wherein o represents from 1 to 400 repeating units.

Along the present invention the compounds of formula I have been named as N-oleoyl-N/O sulfated chitosans (CHIT-X-L_{Y}-N/O-Z), wherein:
X represents M_{w} (average molecular weight of the polysaccharide), which is defined as the total weigh of polysaccharide divided by the total number of molecules;
L represents functionalization of the chitosan by R⁴ as shown in formula **I**, where R⁴ represents oleoyl, being oleoyl = -CO-(CH₂)₇-CH=CH-(CH₂)₇-CH₃.
γ represents the percentage degree of oleoyl functionalization, which is defined as m/(m+n+o)•100 according to formula **I**;
N represents a compound of formula **I** with a sulfatation degree Z, where R¹ is H or - SO₃M, R² is H, and R³ is H;
O represents a compound of formula **I** with a sulfatation degree Z, where R¹ is H, R² is H or -SO₃M, and/or R³ is H or -SO₃M;
Z represents the average number of sulfate groups in a repeating unit, as defined above;and
M represents a monovalent cation.

In another embodiment the invention relates to the compound of formula I as defined above selected from:
m= 1-5, n= 1-5, o= 5-40, R¹= H, R²= SO₃⁻Na⁺ or H, R³= SO₃⁻Na⁺ or H, Z= 1.6 (CHIT-5-L₁₅-O-1.6);
m= 0, n= 1-5, o= 9-35, R¹= SO₃⁻Na⁺ or H, R²= SO₃⁻Na⁺ or H, R³= SO₃⁻Na⁺ or H, Z= 2.5 (CHIT-5-N,O-2.5);
m= 0, n= 1-5, o= 9-35, R¹= H, R²= SO₃⁻Na⁺ or H, R³= SO₃⁻Na⁺ or H, Z= 0.5-2.0 (CHIT-5-O-1.8);
m= 10-20, n= 5-15; o= 55-95, R¹= H, R²= SO₃⁻Na⁺ or H, R³= SO₃⁻Na⁺ or H, Z= 1.6 (CHIT-15-L₁₅O-1.6);
m= 20-35, n= 5-15, o= 40-70, R¹= H, R²= SO₃⁻Na⁺ or H, R³= SO₃⁻Na⁺ or H, Z= 1.7 (CHIT-15-L₃₀-O-1.7);
m= 40-70, n= 5-15, o= 20-35, R¹= H, R²= SO₃⁻Na⁺ or H, R³= SO₃⁻Na⁺ or H, Z= 1.6 (CHIT-15-L₅₀-O-1.6);
m= 10-20, n= 5-15; o= 55-95, R¹= SO₃⁻Na⁺ or H, R²= R³= H, Z= 0.7 (CHIT-15- L₁₅-N-0.7);
m= 0, n= 5-15, o= 65-105, R¹= SO₃⁻Na⁺ or H, R²= SO₃⁻Na⁺ or H, R³= SO₃⁻Na⁺ or H, Z= 2.0 (CHIT-15-N,O-2.0);
m= 0, n= 5-15, o= 65-105, R¹= H, R²= SO₃⁻Na⁺ or H, R³= SO₃⁻Na⁺ or H, Z= 1.6 (CHIT-15-0-1.6);
m= 0, n= 5-15, o= 65-105, R¹= SO₃⁻Na⁺ or H, R²= R³= H, Z= 0.7 (CHIT-15-N-0.7);
m= 7-30, n= 5-20, o= 45-125, R¹= H, R²= SO₃⁻Na⁺ or H, R³= SO₃⁻Na⁺ or H, Z= 1.7 (CHIT-20-L₁₅-O-1.7);
m= 15-35, n= 15-40, o= 75-160, R¹= H, R²= SO₃⁻Na⁺ or H, R³= SO₃⁻Na⁺ or H, Z= 1.7 (CHIT-30-L₁₅-O-1.7);
m= 33-54, n= 33-54, o= 154-252, R¹= H, R²= R³= SO₃⁻Na⁺ or H, Z= 1.6 (CHIT-50-L₁₅-0-1.6)
m= 69-90, n= 92-120, o= 299-390, R¹= H, R²= SO₃⁻Na⁺ or H, R³= SO₃⁻Na⁺ or H, Z= 1.7 (CHIT-90-L₁₅-O-1.7);
m= 105-173, n= 105-173, o= 490-806, R¹= H, R²= SO₃⁻Na⁺ or H, R³= SO₃⁻Na⁺ or H, Z= 1.6 (CHIT-125-L₁₅-O-1.6)

In another embodiment the invention relates to the compound of formula I as defined above selected from:
m= 10-20, n= 5-15; o= 55-95, R¹= H, R²= SO₃⁻Na⁺ or H, R³= SO₃⁻Na⁺ or H, Z= 1.6 (CHIT-15-L₁₅-O-1.6);
m= 20-35, n= 5-15, o= 40-70, R¹= H, R²= SO₃⁻Na⁺ or H, R³= SO₃⁻Na⁺ or H, Z= 1.6 (CHIT-15-L₃₀-O-1.7);
m= 0, n= 5-15, o= 65-105, R¹= SO₃⁻Na⁺ or H, R²= R³= H, Z= 0.7 (CHIT-15-N-0.7);
m= 0, n= 5-15, o= 65-105, R¹= SO₃⁻Na⁺ or H, R²= SO₃⁻Na⁺ or H, R³= SO₃⁻Na⁺ or H,
Z= 2.0 (CHIT-15-N,O-2.0);
m= 0, n= 5-15, o= 65-105, R¹= H, R²= SO₃⁻Na⁺ or H, R³= SO₃⁻Na⁺ or H, Z= 1.6 (CHIT-15-0-1.6);
m= 15-35, n= 15-40, o= 75-160, R¹= H, R²= SO₃⁻Na⁺ or H, R³= SO₃⁻Na⁺ or H, Z= 1.7 (CHIT-30-L₁₅-O-1.7); and
m= 33-54, n= 33-54, o= 154-252, R¹= H, R²= SO₃⁻Na⁺ or H, R³= SO₃⁻Na⁺ or H, Z= 1.6 (CHIT-50-L₁₅-O-1.6).

In another embodiment the invention relates to the compound of formula I as defined above, selected from:
m= 10-20, n= 5-15, o= 55-95, R¹= H, R²= SO₃⁻Na⁺ or H, R³= SO₃⁻Na⁺ or H, Z= 1.7 (CHIT-L₁₅-O-1.6);
m= 20-35, n= 5-15, o= 40-70, R¹= H, R²= SO₃⁻Na⁺ or H, R³= SO₃⁻Na⁺ or H, Z= 1.6 (CHIT-15-L₃₀-O-1.7);
m= 15-35, n= 15-40, o= 75-160, R¹= H, R²= SO₃⁻Na⁺ or H, R³= SO₃⁻Na⁺ or H, Z= 1.7 (CHIT-30-L₁₅-O-1.7); and
m= 33-54, n= 33-54, o= 154-252, R¹= H, R²= SO₃⁻Na⁺ or H, R³= SO₃⁻Na⁺ or H, Z= 1.6 (CHIT-50-L₁₅-O-1.6).

Present invention describes that sulfated oleoyl-chitosan derivatives act as mimetics of natural HSs, acting as a decoy trap, or what is the same, preventing the virus from adhering to epithelial cells, thus slowing down the process of infection. In this way, the use of these derivatives could be a therapeutic solution for SARS-CoV-2, causative agent of the COVID-19 pandemic, for respiratory syncytial virus (RSV) and, among others, for MERS, SARS-CoV-1, human Metapneumovirus (hMPV), Ebola Virus (EBOV), Herpes Simplex Virus (HSV), Human papillomavirus (HPV) and Dengue Virus (DENV).

Another aspect of the present invention relates to a pharmaceutical composition comprising a compound of formula I as defined above or a pharmaceutically acceptable salt thereof, together with a pharmaceutically acceptable vehicle, and optionally another active substance.

Another aspect of the present invention relates to a compound of formula I as defined above or a pharmaceutical acceptable salt thereof, for use in therapy.

Another aspect of the present invention relates to a compound of formula I as defined above or a pharmaceutical acceptable salt thereof, for use in the treatment and/or prevention of a viral infection, preferably wherein the viral infection is caused by HSPG-dependent viruses (SARS-CoV-2, SARS-CoV-1, MERS, Ebola Virus (EBOV), Herpes Simplex Virus (HSV), Human papillomavirus (HPV), Dengue Virus (DENV), Human Metapneumovirus or respiratory syncytial virus (RSV)), more preferably wherein the viral infection is caused by SARS-CoV-2 or RSV.

Another aspect of the present invention relates to the use of a compound of formula I or a pharmaceutically acceptable salt thereof for the manufacture of a medicament for the treatment and/or prevention of a viral infection, preferably wherein the viral infection is caused by SARS-CoV-2, SARS-CoV-1, MERS, respiratory syncytial virus (RSV), Ebola Virus (EBOV), human Metapneumovirus (hMPV), Herpes Simplex Virus (HSV), Human papillomavirus (HPV) and Dengue Virus (DENV), among others; more preferably wherein the viral infection is caused by SARS-CoV-2 or by RSV.

Another aspect of the present invention relates to a method of treating a viral infection, preferably wherein the viral infection is caused by SARS-CoV-2, respiratory syncytial virus (RSV), SARS-CoV-1, MERS, human Metapneumovirus (hMPV), Ebola Virus (EBOV), Herpes Simplex Virus (HSV), Human papillomavirus (HPV) and Dengue Virus (DENV), among others; more preferably wherein the viral infection is caused by SARS-CoV-2 or by RSV, in a subject in need thereof, especially a human being, which comprises administering to said subject an effective amount of a compound of formula I or a pharmaceutically acceptable salt thereof.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### DESCRIPTION OF THE DRAWINGS

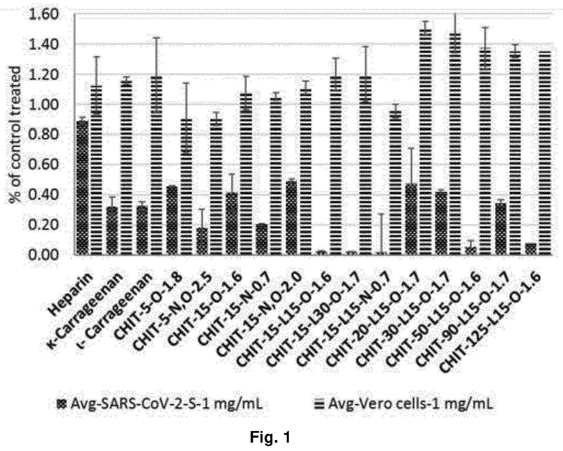

**Fig. 1****. Pre infection drug treatment.** Shows the effect of pretreating SARS-CoV-2 Spike protein pseudotyped vesicular stomatitis virus (VSV) with the drugs prior to infection. Pseudotyped vesicular stomatitis virus reporter viruses (encoding GFP and firefly luciferase) carrying the S protein of SARS-CoV-2 (VSV-S) were pre-incubated with the indicated compound and concentration for 1 hour at 37 °C, or mock treated with the diluent alone. Subsequently, these were used to infect Vero cells in a 96 well plate for 16 hours, at which point virus infection was quantified by examining luciferase signal. The average luciferase signal was standardized relative to the mock treated condition. Plotted is the average standardized luciferase signal relative to mock treatment of 3 replicates at 1 mg/mL (Avg-1 mg/mL) or 200 µg/mL (Avg-200 µg/mL) or the average cell viability (Avg-viability).

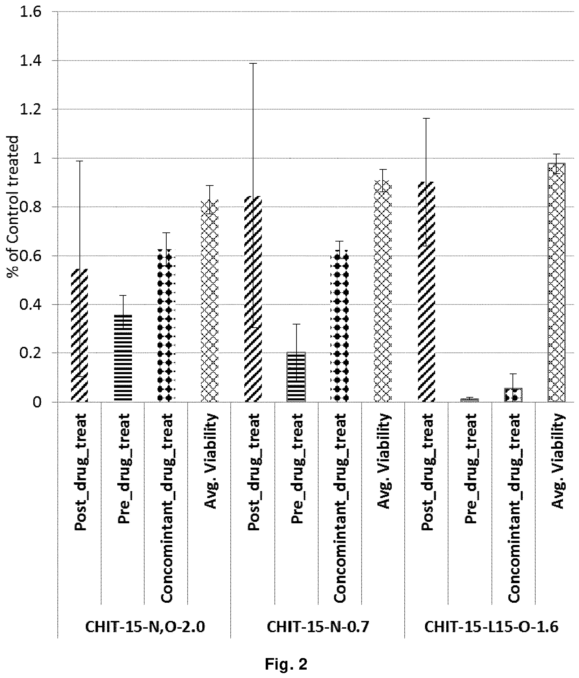

**Fig. 2****. Pre, concomitant, and post infection drug treatment.** Shows the antiviral effect of the drugs when added at different stages of the infection process. Three conditions were used to assess the time at which the drugs are acting: preincubation of pseudotyped vesicular stomatitis virus reporter viruses (encoding GFP and luciferase) carrying the S protein of SARS-CoV-2 (VSV-S) for 1 hour at 37 °C prior to infection of Vero cells, pretreatment of Vero cells with the indicated compounds for 1 hour at 37 °C prior to infection with VSV-S, or concomitant infection and drug treatment. A corresponding mock treatment with the diluent alone was performed for each condition. After 16 hours, virus infection was quantified by examining luciferase signal in the cells. The average luciferase signal was standardized relative to the mock treated condition. Plotted is the average standardized luciferase signal relative to mock treatment of 3 replicates as well as the standard deviation.

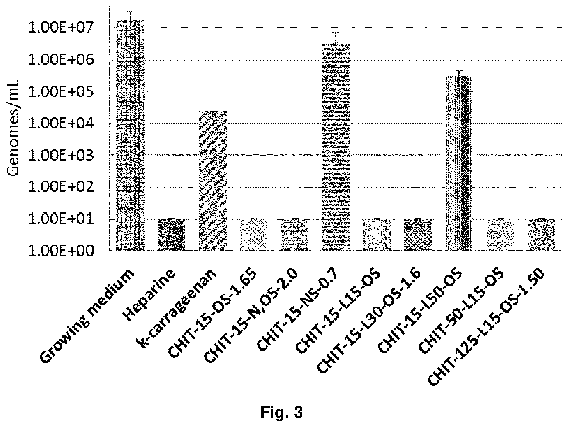

**Fig 3****. Pretreatment of SARS-CoV-2 prior to infection.** Shows the effect of pretreating SARS-CoV-2 with the drugs on infection. The virus was amplified at a multiplicity of infection of 0.001 for 60 hours on Vero cells and tittered via limiting dilution. For antiviral assays, 10⁴ 50% Tissue Culture Infectious Dose (TCid50) of the virus were premixed with the compounds for 1 hour at 37 °C. Subsequently, the mixture was used to infect Vero cells in a 24-well plate for 1 hour at 37 °C, after which the inoculum was removed and media containing the compound added. Virus production was assessed by qRT-PCR following 24 using the N1 primers of the CDC assay.

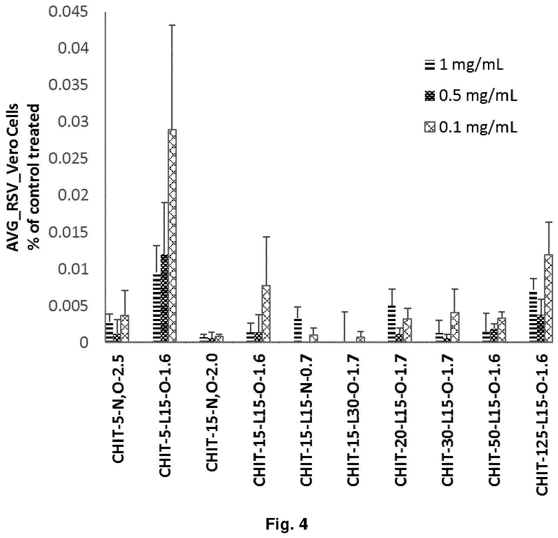

**Fig 4****. Pretreatment of RSV prior to infection of Vero cells.** Shows the effect of pretreating respiratory syncytial virus (RSV; strain A2line19F-mKate encoding red fluorescent protein mKate) with the drugs prior to infection. RSV was pre-incubated with the indicated compound and concentration for 1 hour at 37 °C, or mock treated with the diluent alone. Subsequently, Vero cells were infected with the drug/virus mixture in a 96 well plate for 24 hours, at which point virus infection was quantified by examining red fluorescence signal. The average fluorescence signal was standardized relative to the mock treated condition. Plotted is the average standardized fluorescence signal relative to mock treatment of 3 replicates as well as the standard deviation.

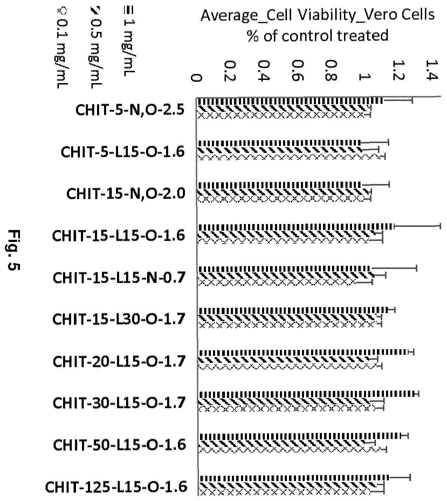

**Fig 5****. Cytotoxic activity against Vero cells.** Shows the effect the drugs on cell viability. Vero cells were treated with the indicated drug or diluent at the indicated concentration in a 96 well plate for 24 hours. Cell viability was assessed by adding resazurin (Sigma-Aldrich, R7017) to a final concentration of 0.4 mg/mL, incubation for 1 hour at 37°C, and reading fluorescence read on a Tecan Spark microplate reader with an excitation of 535 nm and emission of 595 nm. The fluorescent signal from drug treated cells was standardized to the mock treated cells. The average fluorescence signal was standardized relative to the mock treated condition. Plotted is the average standardized fluorescence signal relative to mock treatment of 3 replicates as well as the standard deviation.

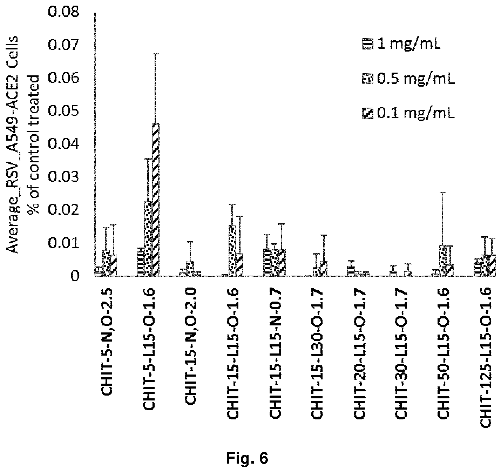

**Fig 6****. Pretreatment of RSV prior to infection of A549-ACE2 cells.** Shows the effect of pretreating respiratory syncytial virus (RSV, strain A2line19F-mKate encoding red fluorescent protein mKate) with the drugs prior to infection. RSV was pre-incubated with the indicated compound and concentration for 1 hour at 37 °C, or mock treated with the diluent alone. Subsequently, A549-ACE2 cells were infected with the drug/virus mixture in a 96 well plate for 24 hours, at which point virus infection was quantified by examining red fluorescence signal. The average fluorescence signal was standardized relative to the mock treated condition. Plotted is the average standardized fluorescence signal relative to mock treatment of 3 replicates as well as the standard deviation.

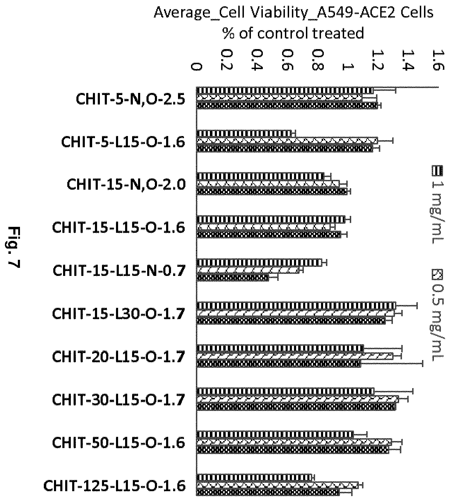

**Fig 7****. Cytotoxic activity against A549-ACE2 cells.** Shows the effect the drugs on cell viability. A549-ACE2 cells were treated with the indicated drug or diluent at the indicated concentration in a 96 well plate for 24 hours. Cell viability was assessed by adding resazurin (Sigma-Aldrich, R7017) to a final concentration of 0.4 mg/mL, incubation for 1 hour at 37 °C, and reading fluorescence read on a Tecan Spark microplate reader with an excitation of 535 nm and emission of 595 nm. The fluorescent signal from drug treated cells was standardized to the mock treated cells. The average fluorescence signal was standardized relative to the mock treated condition. Plotted is the average standardized fluorescence signal relative to mock treatment of 3 replicates as well as the standard deviation.

### Examples

### 1. STARTING CHITOSANS

| **SAMPLE**^{[a]} | **Mw** (KDa) | **DDA**^{[d]} | **Supplier** | |
|---|---|---|---|---|
| ***CHIT-5*** | ≤ 5^{[b]} | 87%^{[e]} | Glentham Sciences | Life |
| ***CHIT-15*** | ≈ 15^{[c]} | 90%^{[e]} | *Homemade preparation*^{[f]} | |
| ***CHIT-20*** | ≈ 20^{[b]} | 90%^{[e]} | Glentham Sciences | Life |
| ***CHIT-30*** | ≈ 30^{[b]} | 84%^{[e]} | Glentham Sciences | Life |
| ***CHIT-50*** | ≈ 50^{[b]} | 85%^{[e]} | Glentham Sciences | Life |
| ***CHIT-150*** | ≈ 190 -310^{[b]} | 85%^{[e]} | Sigma-Aldrich | |

[a] The starting samples are named according to their molecular weight (CHIT-X (Mw(KDa))); [b] Data provided by the supplier; [c] Data measured by GPC; [d] Deacetylation degree; [e] Values estimated by ¹H NMR (400 MHz, 70 °C): Degree of Deacetylation (DDA) (%)= 1-[(1/3H_{Ac})/(1/6H₂₋₆)]×100 where H_{Ac} is the integration value of the three protons of the acetyl group (≈ 2.00 ppm) and H₂₋₆ the integration value for signals from protons (H2, H3, H4, H5, H6 and H6') of the glucosamine (≈ 3.5 - 4.4 ppm). [f] CHIT-15 was prepared according to the procedure described below.

### Preparation of starting material CHIT-15

Starting chitosan was provided by Idebio, S.L. (Mw= 150 kDa, Degree of Deacetylation (DDA)= 87%). Chitosan (10 g) was solved in a 0.1N aqueous solution of HCI (660 mL) and was stirred overnight at room temperature. The solution was heated to 37 °C in an orbital shaker (100 rpm). In parallel, KNO₂ (66.7 mg) was solved in an aqueous solution of HCI (0.1 N) (25 mL) at 37 °C in a dark bottle, on which is poured the chitosan solution. The mixture was reacted for 15 min, and then chitosan was precipitated with an aqueous solution of NaOH (50% w/v) until pH= 8. The precipitate was washed with mixtures of H₂O/EtOH (80:20; 90:10; 100:0). Finally, the solid was dried at 60 °C and analyzed by GPC.
¹H NMR (D₂O, 500 MHz, 25 ºC, TFA (1%)) (selected signals): H₃C-CONH- (1.96 ppm); H₂ (3.06 ppm); H₂₋₅ (3.49-3.99 ppm).
M_{w} (kDa)= 15.0

### 2. GENERAL PROCEDURES

### 2.1. Procedure 1: Oleic-acid grafted chitosan

Oleic-acid grafted chitosan was synthesized via the reaction of the amino group of chitosan with the carboxyl group of oleic acid. Briefly, 1.0 g of chitosan (**CHIT-X**) was dissolved in a solution mixture of 100 mL of 1% (v/v) acetic acid water solution and was stirred at room temperature for 15 hours. Then, 85 mL of methanol was added and the mixture was stirred for an additional hour. In parallel, oleic acid (OA) (0.57 mL) was reacted with 1-ethyl-3-(3-dimethylaminopropyl)carbodiimide (EDC) (0.17 g) in a methanol solution (15 mL) for 2 hours at room temperature after which the obtained solution was poured into the chitosan solution. After being allowed to react for 18 h, the mixture was dialyzed firstly against a solution of ethanol in water (10% v/v) for 18 hours and, finally, against distilled water for 2 days. The obtained solution was lyophilized to obtain oleic-acid grafted chitosan **(CHIT-X-L_{y}).**

In order to obtain derivatives with different degree of substitution, reactions with different chitosan-EDC-oleic acid ratios were carried out.

### 2.2. Procedure 2: O-sulfation of chitosan

A solution of chitosan (**CHIT-X**) or oleic-acid grafted chitosan (**CHIT-X-L_{y}**) (0.5 g) in formic acid (23 mL) was stirred within 1 h at room temperature. Subsequently, dimethlyformamide (DMF) (122 mL) was added and the mixture was stirred during another 2 h. In parallel, a solution of chlorosulfonic acid (1.4 mL) in DMF (8 mL) was prepared. This mixture was added dropwise into the chitosan (**CHIT-X** or **CHIT-X-L_{Y}**) solution within 15 min, and the mixture was kept at room temperature for 5 h. The resulting yellow solution was added into a saturated solution of anhydrous sodium acetate in ethanol (300 mL), and the mixture was kept overnight. The obtained precipitate was washed with an ethanol/water (4:1) mixture (v/v) and then dissolved in water (45 mL). The pH of the obtained solution was increased until pH 7.5 with 1 M NaOH. Finally, the solution was concentrated to one-fourth of the original volume and then dialyzed against deionized water and freeze-dried to obtain O-sulfated chitosan (**CHIT-X-O-Z**) or oleic-acid grafted O-sulfated chitosan **(CHIT-X-L_{y}-O-Z).**

The application of this process allows to achieve a degree of *O*-sulfation of about 1.5-1.8, depending on the starting material used. To obtain derivatives with different degrees of sulfation, reactions with different chitosan-chlorosulfonic acid ratios were carried out.

### 2.3. Procedure 3: CHIT N-sulfation

Chitosan (**CHIT-X**), O-sulfated chitosan (**CHIT-X-O-Z**) or oleic-acid grafted chitosan **(CHIT-X-L_{y})** (1.0 g) was dispersed in deionized water (30 mL), and Na₂CO₃ (357 mg) and Me₃N·SO₃ (710 mg) were added. The obtained suspension was heated at 50 °C until a yellow viscous solution was formed (~24 h). The mixture was cooled, dialyzed exhaustively against Milli-Q water, and then lyophilized to give N-sulfated chitosan (**CHIT-X-N-Z**) N,O-sulfated chitosan (**CHIT-X-N,O-Z**) or oleic-acid grafted N-sulfated chitosan **(CHIT-X-L_{y}-N-Z).**

The application of this process allows to achieve a degree of *N*-sulfation of about 1.5-1.8, depending on the starting material used. To obtain derivatives with different degrees of sulfation, reactions with different chitosan-chlorosulfonic acid ratios were carried out.

In order to obtain derivatives with different degree of sulfation, reactions with different chitosan-Na₂CO₃-Me₃N·SO₃ ratios were carried out.

### 3. EXAMPLES

### 3.1. Synthesis of CHIT-X-L_{Y}-O-Z polysaccharides 3.1.1. CHIT-5-L₁₅-O-1.6 (X=5; Y=15; Z= 1.6)

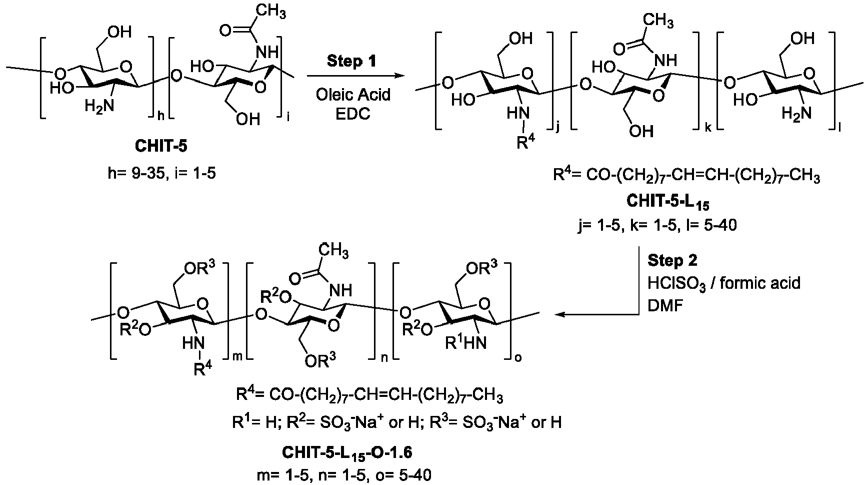

### Step 1: CHIT-5 was grafted with oleic acid according to Procedure 1, using a CHIT-5:EDC:oleic acid ratio of 1:0.17:0.57 (g:g:mL). Yield of CHIT-5-L₁₅: 53%.

¹H NMR (D₂O, 500 MHz, 25 ºC, TFA (1%)) (selected signals): -CO-CH₂-(CH₂)₅-CH₂-CH=CH-CH₂-(CH₂)₆-CH₃ (0.88 ppm); -CO-CH₂-(CH₂)₅-CH₂-CH=CH-CH₂-(CH₂)₆-CH₃ (1.22-1.53 ppm); -CO-CH₂-(CH₂)₅-CH₂-CH=CH-CH₂-(CH₂)₆-CH₃ (1.97-2.0 and 2.19-2.28); H₃C-CONH- (2.07 ppm); H₂₋₆ (2.6-4.7 ppm); H₁ (4.6-4.8 ppm) and -CO-CH₂-(CH₂)₅-CH₂-CH=CH-CH₂-(CH₂)₆-CH₃ (5.45 ppm).

### Step 2: CHIT-5-L₁₅-O-1.6 was prepared from CHIT-5-L₁₅ according to Procedure 2, using a CHIT-5-L₁₅:chlorosulfonic acid ratio of 1:2.8 (g:mL). Yield of CHIT-5-L₁₅-O-1.6: 98%

**¹H NMR** (D₂O, 500 MHz, 25 ºC) (selected signals): -CO-CH₂-(CH₂)₅-CH₂-CH=CH-CH₂-(CH₂)₆-CH₃ (0.88 ppm); -CO-CH₂-(CH₂)₅-CH₂-CH=CH-CH₂-(CH₂)₆-CH₃ (1.22-1.53 ppm); -CO-CH₂-(CH₂)₅-CH₂-CH=CH-CH₂-(CH₂)₆-CH₃ (1.97-2.0 and 2.19-2.28); H₃C-CONH- (2.07 ppm); H₂ (2.97 ppm); H₃(H) and H_{6a-b} (H) (3.6-4.0 ppm); H₃ (SO₃⁻) and H_{6a-b} (SO₃⁻) (4.0-4.5 ppm).

**Elemental analysis:** %S= 12.43; %N= 2.96; S/N= 1.6.

### 3.1.2. CHIT-15-L₁₅-O-1.6 (X=15; Y=15; Z= 1.6)

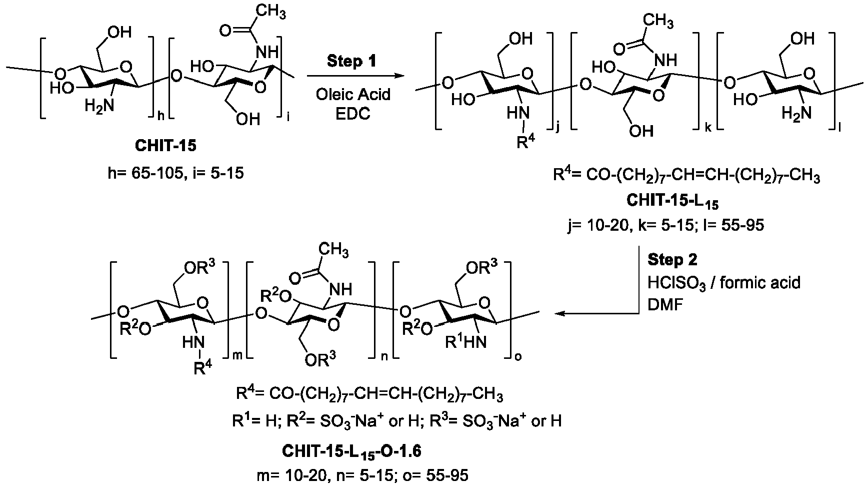

### Step 1: CHIT-15 was grafted with oleic acid according to Procedure 1 , using a CHIT-15:EDC:oleic acid ratio of 1:0.17:0.57 (g/g/mL). Yield of CHIT-15-L₁₅: 85%.

**¹H NMR** (D₂O, 500 MHz, 25 ºC, TFA (1%)) (selected signals): -CO-CH₂-(CH₂)₅-CH₂-CH=CH-CH₂-(CH₂)₆-CH₃ (0.58 ppm); -CO-CH₂-(CH₂)₅-CH₂-CH=CH-CH₂-(CH₂)₆-CH₃ (0.98-1.25 ppm); -CO-CH₂-(CH₂)₅-CH₂-CH=CH-CH₂-(CH₂)₆-CH₃ (1.61-2.01); H₃C-CONH- (1.95 ppm); H₂ (2.85 ppm); H₃₋₆ (3.27-3.76 ppm) and H₁ (4.99 ppm).

### Step 2: CHIT-15-L₁₅-O-1.6 was prepared from CHIT-15-L₁₅ according to Procedure 2 using a CHIT-15-L₁₅:chlorosulfonic acid ratio of 1:2.8 (g:mL). Yield of CHIT-15-L₁₅-O-1.6: 96%

**¹H NMR** (D₂O, 500 MHz, 25 ºC) (selected signals): -CO-CH₂-(CH₂)₅-CH₂-CH=CH-CH₂-(CH₂)₆-CH₃ (0.77 ppm); -CO-CH₂-(CH₂)₅-CH₂-CH=CH-CH₂-(CH₂)₆-CH₃ (1.07-1.55 ppm); -CO-CH₂-(CH₂)₅-CH₂-CH=CH-CH₂-(CH₂)₆-CH₃(1.86-1.93 and 2.23-2.31); H₃C-CONH- (1.95 ppm); H₂ (3.07 ppm); H₃(H) and H_{6a-b} (H) (3.23-3.77 ppm); H₃ (SO₃⁻) and H_{6a-b} (SO₃⁻) (3.85-4.55 ppm); H₁ (4.87 ppm) and -CO-CH₂-(CH₂)₅-CH₂-CH=CH-CH₂-(CH₂)₆-CH₃ (5.81 ppm).

**Elemental analysis:** %S= 12.43; %N= 2.96; S/N= 1.6.

### 3.1.3. CHIT-15-L₃₀-O-1.7 (X= 15; Y= 30; Z= 1.7).

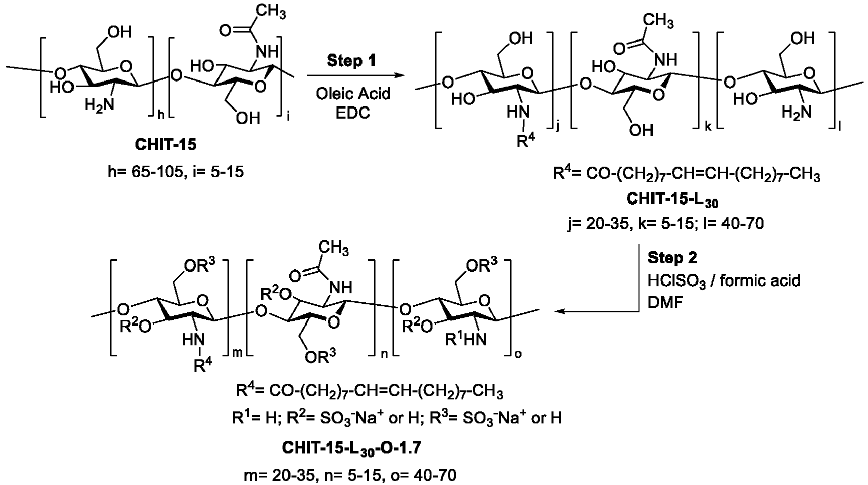

### Step 1: CHIT-15 was grafted with oleic acid according to Procedure 1 , using a CHIT-15:EDC:oleic acid ratio of 1:0.34:1.14 (g:g:mL). Yield of CHIT-15-L₃₀: 70%.

**¹H NMR** (D₂O, 500 MHz, 25 ºC, TFA (1%)) (selected signals): -CO-CH₂-(CH₂)₅-CH₂-CH=CH-CH₂-(CH₂)₆-CH₃ (0.55 ppm); -CO-CH₂-(CH₂)₅-CH₂-CH=CH-CH₂-(CH₂)₆-CH₃ (0.97-1.26 ppm); -CO-CH₂-(CH₂)₅-CH₂-CH=CH-CH₂-(CH₂)₆-CH₃ (1.59-1.80); H₃C-CONH- (1.97 ppm); H₂ (2.86 ppm); H₃₋₆ (3.32-3.68 ppm) and H₁ (5.02 ppm).

### Step 2: CHIT-15-L₃₀-O-1.7 was prepared from CHIT-15-L₃₀ according to Procedure 2, using a CHIT-15-L₃₀:chlorosulfonic acid ratio of 1:2.8 (g:mL)Yield of CHIT-15-L₃₀-O-1.7: 96%

**¹H NMR** (D₂O, 500 MHz, 25 ºC) (selected signals): -CO-CH₂-(CH₂)₅-CH₂-CH=CH-CH₂-(CH₂)₆-CH₃ (0.77 ppm); -CO-CH₂-(CH₂)₅-CH₂-CH=CH-CH₂-(CH₂)₆-CH₃ (0.96-1.62 ppm); -CO-CH₂-(CH₂)₅-CH₂-CH=CH-CH₂-(CH₂)₆-CH₃(1.83-1.99 and 2.13-2.34); H₃C-CONH- (2.07 ppm); H₂ (3.04 ppm); H₃(H) and H_{6a-b} (H) (3.12-3.46 ppm) and H₃ (SO₃⁻) and H_{6a-b} (SO₃⁻) (3.68-4.37 ppm).

**Elemental analysis:** %S= 13.54; %N= 3.52; S/N= 1.7.

### 3.1.4. CHIT-15-L₅₀-O-1.6 (X= 15; Y= 50; Z= 1.6).

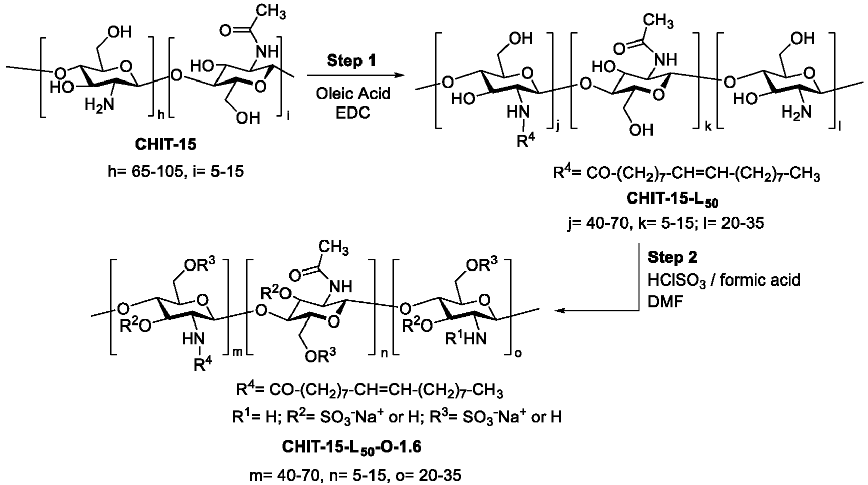

### Step 1: CHIT-15 was grafted with oleic acid according to Procedure 1 using a CHIT-15:EDC:oleic acid ratio of 1:0.68:2.28 (g:g:mL) Yield of CHIT-15-L₅₀: 69%.

**¹H NMR** (D₂O, 500 MHz, 25 ºC, TFA (1%)) (selected signals): -CO-CH₂-(CH₂)₅-CH₂-CH=CH-CH₂-(CH₂)₆-CH₃ (0.58 ppm); -CO-CH₂-(CH₂)₅-CH₂-CH=CH-CH₂-(CH₂)₆-CH₃ (0.98-1.25 ppm); -CO-CH₂-(CH₂)₅-CH₂-CH=CH-CH₂-(CH₂)₆-CH₃ (1.61-2.01); H₃C-CONH- (1.95 ppm); H₂ (2.85 ppm); H₃₋₆ (3.27-3.76 ppm) and H₁ (4.99 ppm).

### Step 2: CHIT-15-L₅₀-O-1.6 was prepared from CHIT-15-L₅₀ according to Procedure 2, using a CHIT-15-L₅₀:chlorosulfonic acid ratio of 1:2.8 (g:mL). Yield of CHIT-15-L₅₀-O-1.6: 65%

**¹H NMR** (D₂O, 500 MHz, 25 ºC) (selected signals): -CO-CH₂-(CH₂)₅-CH₂-CH=CH-CH₂-(CH₂)₆-CH₃ (0.77 ppm); -CO-CH₂-(CH₂)₅-CH₂-CH=CH-CH₂-(CH₂)₆-CH₃ (1.07-1.55 ppm); -CO-CH₂-(CH₂)₅-CH₂-CH=CH-CH₂-(CH₂)₆-CH₃ (1.86-1.93 and 2.23-2.31); H₃C-CONH- (1.95 ppm); H₂ (3.07 ppm); H₃(H) and H_{6a-b} (H) (3.23-3.77 ppm); H₃ (SO₃⁻) and H_{6a-b} (SO₃⁻) (3.85-4.55 ppm); H₁ (4.87 ppm) and -CO-CH₂-(CH₂)₅-CH₂-CH=CH-CH₂-(CH₂)₆-CH₃ (5.81 ppm).

**Elemental analysis:** %S= 12.43; %N= 2.96; S/N= 1.6.

### 3.1.5. CHIT-20-L₁₅-O-1.7 (X= 20; Y= 15; Z= 1.7).

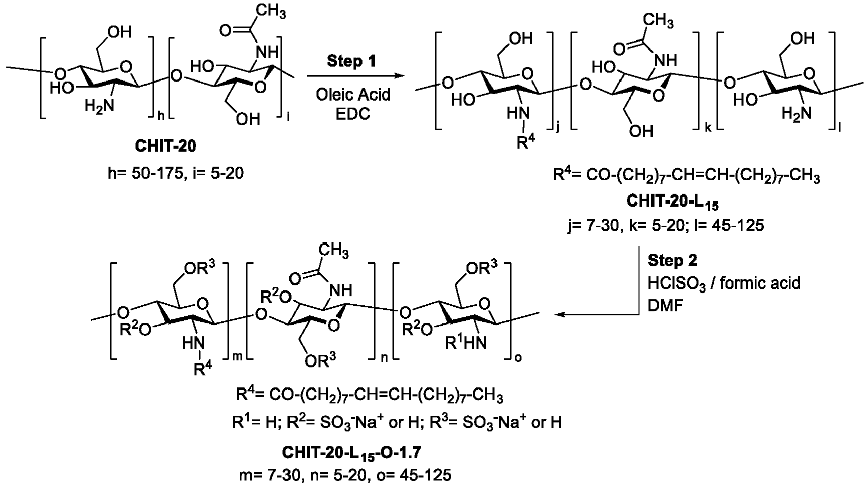

### Step 1: CHIT-20 was grafted with oleic acid according to Procedure 1, using a CHIT-20:EDC:oleic acid ratio of 1:0.17:0.57 (g:g:mL). Yield of CHIT-20-L₁₅: 66%.

**¹H NMR** (D₂O, 500 MHz, 25 ºC, TFA (1%)) (selected signals): -CO-CH₂-(CH₂)₅-CH₂-CH=CH-CH₂-(CH₂)₆-CH₃ (0.71 ppm); -CO-CH₂-(CH₂)₅-CH₂-CH=CH-CH₂-(CH₂)₆-CH₃ (1.05-1.47 ppm); -CO-CH₂-(CH₂)₅-CH₂-CH=CH-CH₂-(CH₂)₆-CH₃ (1.80-1.89 and 2.03-2.11); H₃C-CONH- (1.90 ppm); H₂ (3.00 ppm) H₃₋₆ (3.42-3.86 ppm); H₁ (4.67 ppm) and -CO-CH₂-(CH₂)₅-CH₂-CH=CH-CH₂-(CH₂)₆-CH₃ (5.13 ppm).

### Step 2: CHIT-20-L₁₅-O-1.7 was prepared from CHIT-20-L₁₅ according to Procedure 2, using a CHIT-20-L₁₅:chlorosulfonic acid ratio of 1:2.8 (g:mL). Yield of CHIT-20-L₁₅-O-1.7: 99%

**¹H NMR** (D₂O, 500 MHz, 25 ºC) (selected signals): -CO-CH₂-(CH₂)₅-CH₂-CH=CH-CH₂-(CH₂)₆-CH₃ (0.73 ppm); -CO-CH₂-(CH₂)₅-CH₂-CH=CH-CH₂-(CH₂)₆-CH₃ (1.03-1.51 ppm); -CO-CH₂-(CH₂)₅-CH₂-CH=CH-CH₂-(CH₂)₆-CH₃ (1.89-1.94); H₃C-CONH-(1.95 ppm); H₂ (3.06 ppm); H₃(H) and H_{6a-b} (H) (3.2-3.98 ppm); H₃ (SO₃⁻) and H_{6a-b} (SO₃⁻) (4.00-4.60 ppm).

**Elemental analysis:** %S= 14.00; %N= 3.66; S/N= 1.7.

### 3.1.6. CHIT-30-L₁₅-O-1.7 (X= 30; Y= 15; Z= 1.7)

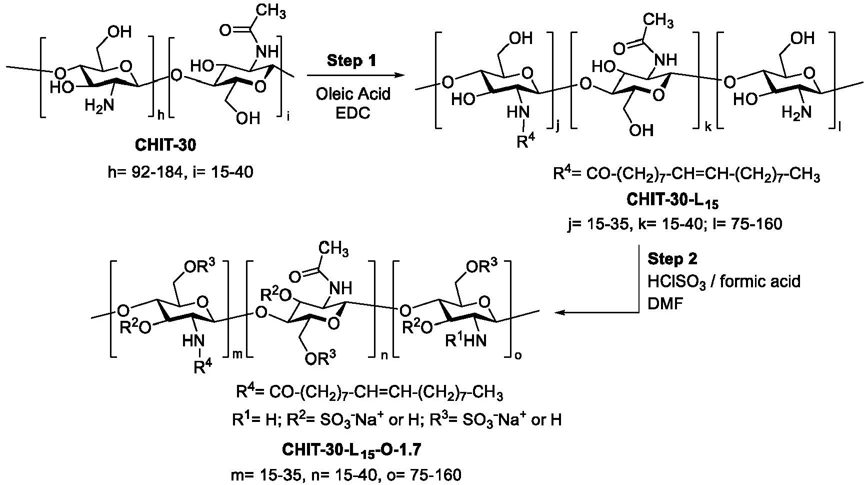

### Step 1: CHIT-30 was grafted with oleic acid according to Procedure 1, using a CHIT-30:EDC:oleic acid ratio of 1:0.17:0.57 (g:g:mL). Yield of CHIT-30-L₁₅: 72%.

**¹H NMR** (D₂O, 500 MHz, 25 ºC, TFA (1%)) (selected signals): -CO-CH₂-(CH₂)₅-CH₂-CH=CH-CH₂-(CH₂)₆-CH₃ (0.78 ppm); -CO-CH₂-(CH₂)₅-CH₂-CH=CH-CH₂-(CH₂)₆-CH₃ (1.07-1.56 ppm); -CO-CH₂-(CH₂)₅-CH₂-CH=CH-CH₂-(CH₂)₆-CH₃ (1.87-1.96 and 2.10-2.19); H₃C-CONH- (1.95 ppm); H₂ (3.06 ppm) H₃₋₆ (3.45-3.79 ppm); H₁ (4.87 ppm) and -CO-CH₂-(CH₂)₅-CH₂-CH=CH-CH₂-(CH₂)₆-CH₃ (5.20 ppm).

### Step 2: CHIT-30-L₁₅-O-1.7 was prepared from CHIT-20-L₁₅ according to Procedure 2, using a CHIT-30-L₁₅:chlorosulfonic acid ratio of 1:2.8 (g:mL). Yield of CHIT-30-L₁₅-O-1.7: 99%

**¹H NMR** (D₂O, 500 MHz, 25 ºC) (selected signals): -CO-CH₂-(CH₂)₅-CH₂-CH=CH-CH₂-(CH₂)₆-CH₃ (0.74 ppm); -CO-CH₂-(CH₂)₅-CH₂-CH=CH-CH₂-(CH₂)₆-CH₃ (1.03-1.53 ppm); H₃C-CONH- (1.92 ppm); H₂ (3.02 ppm); H₃(H) and H_{6a-b} (H) (3.20-3.99 ppm); H₃ (SO₃⁻) and H_{6a-b} (SO₃⁻) (4.01-4.59 ppm); H₁ (4.89 ppm).

**Elemental analysis:** %S= 14.42; %N= 3.73; S/N= 1.7.

### 3.1.7. CHIT-50-L₁₅-O-1.6 (X= 50; Y= 15; Z= 1.6)

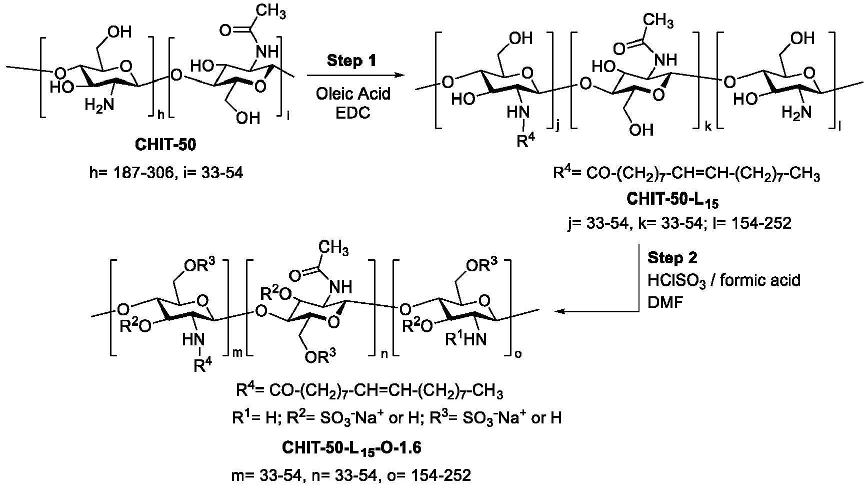

### Step 1: CHIT-50 was grafted with oleic acid according to Procedure 1, using a CHIT-50:EDC:oleic acid ratio of 1:0.17:0.57 (g:g:mL). Yield of CHIT-50-L₁₅: 78%.

**¹H NMR** (D₂O, 500 MHz, 25 ºC, TFA (1%)) (selected signals): -CO-CH₂-(CH₂)₅-CH₂-CH=CH-CH₂-(CH₂)₆-CH₃ (0.78 ppm); -CO-CH₂-(CH₂)₅-CH₂-CH=CH-CH₂-(CH₂)₆-CH₃ (1.07-1.56 ppm); -CO-CH₂-(CH₂)₅-CH₂-CH=CH-CH₂-(CH₂)₆-CH₃ (1.83-1.89 and 2.06-2.15); H₃C-CONH- (1.92 ppm); H₂ (3.01 ppm) H₃₋₆ (3.46-3.86 ppm); H₁ (4.71 ppm) and -CO-CH₂-(CH₂)₅-CH₂-CH=CH-CH₂-(CH₂)₆-CH₃ (5.16 ppm).

### Step 2: CHIT-50-L₁₅-O-1.6 was prepared from CHIT-50-L₁₅ according to Procedure 2, using a CHIT-50-L₁₅:chlorosulfonic acid ratio of 1:2.8 (g:mL). Yield of CHIT-50-L₁₅-O-1.6: 96%

**¹H NMR** (D₂O, 500 MHz, 25 ºC) (selected signals): -CO-CH₂-(CH₂)₅-CH₂-CH=CH-CH₂-(CH₂)₆-CH₃ (0.73 ppm); -CO-CH₂-(CH₂)₅-CH₂-CH=CH-CH₂-(CH₂)₆-CH₃ (1.03-1.55 ppm); H₃C-CONH- (2.00 ppm); H₂ (3.10 ppm); H₃(H) and H_{6a-b} (H) (3.40-3.89 ppm); H₃ (SO₃⁻) and H_{6a-b} (SO₃⁻) (3.95-4.47 ppm).

**Elemental analysis:** %S= 14.88; %N= 3.75; S/N= 1.6.

### 3.1.8. CHIT-90-L₁₅-O-1.7 (X= 90; Y= 15; Z= 1.7)

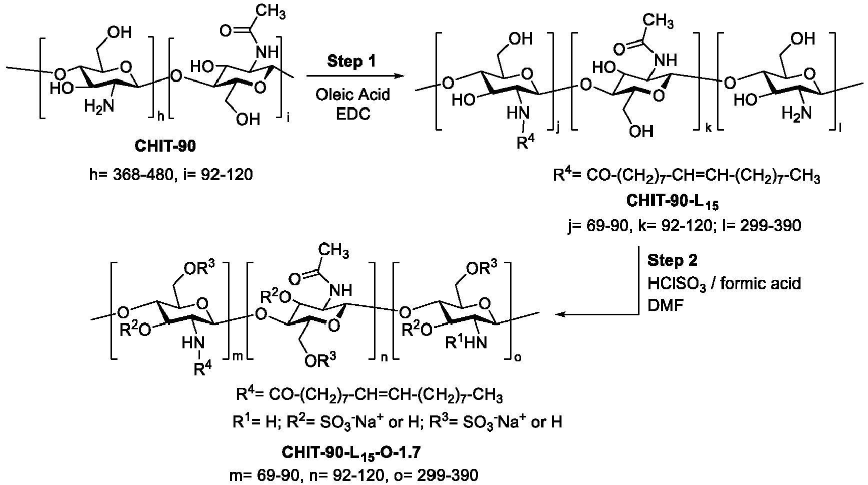

### Step 1: CHIT-90 was grafted with oleic acid according to Procedure 1 , using a CHIT-90:EDC:oleic acid ratio of 1:0.17:0.57 (g:g:mL). Yield of CHIT-90-L₁₅ (Intermediate 1): 83%.

**¹H NMR** (D₂O, 500 MHz, 25 ºC, TFA (1%)) (selected signals): -CO-CH₂-(CH₂)₅-CH₂-CH=CH-CH₂-(CH₂)₆-CH₃ (0.77 ppm); -CO-CH₂-(CH₂)₅-CH₂-CH=CH-CH₂-(CH₂)₆-CH₃ (1.03-1.52 ppm); -CO-CH₂-(CH₂)₅-CH₂-CH=CH-CH₂-(CH₂)₆-CH₃ (1.88-1.94 and 2.11-2.22); H₃C-CONH- (1.96 ppm); H₂ (3.07 ppm) H₃₋₆ (3.48-3.89 ppm); H₁ (4.86 ppm) and -CO-CH₂-(CH₂)₅-CH₂-CH=CH-CH₂-(CH₂)₆-CH₃ (5.22 ppm).

### Step 2: CHIT-90-L₁₅-O-1.7 was prepared from CHIT-90-L₁₅ according to Procedure 2, using a CHIT-90-L₁₅:chlorosulfonic acid ratio of 1:2.8 (g:mL). Yield of CHIT-90-L₁₅-O-1.7: 92%

**¹H NMR** (D₂O, 500 MHz, 25 ºC) (selected signals): -CO-CH₂-(CH₂)₅-CH₂-CH=CH-CH₂-(CH₂)₆-CH₃ (0.73 ppm); -CO-CH₂-(CH₂)₅-CH₂-CH=CH-CH₂-(CH₂)₆-CH₃ (1.03-1.55 ppm); H₃C-CONH- (1.91 ppm); H₂ (3.22 ppm); H₃(H) and H_{6a-b} (H) (3.53-3.98 ppm); H₃ (SO₃⁻) and H_{6a-b} (SO₃⁻) (4.00-4.55 ppm).

**Elemental analysis:** %S= 14.29; %N= 3.74; S/N= 1.7.

### 3.1.9. CHIT-125-L₁₅-O-1.6 (X= 125; Y= 15; Z= 1.6)

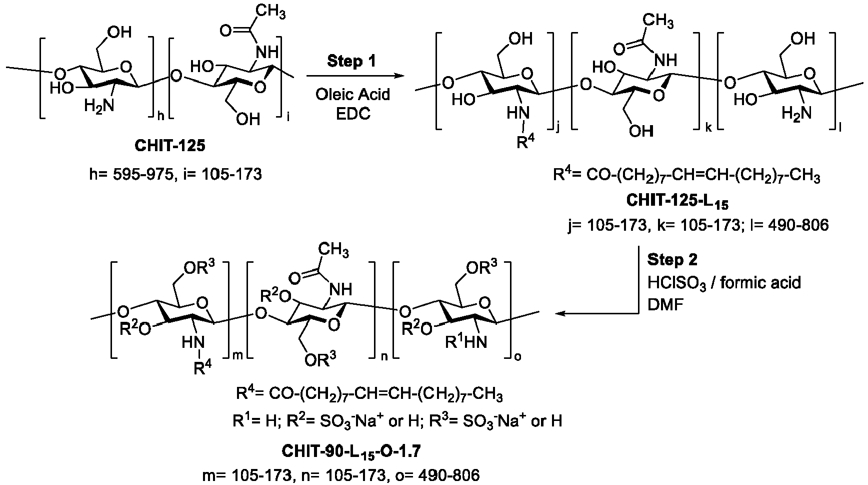

### Step 1: CHIT-125 was grafted with oleic acid according to Procedure 1, using a CHIT-125:EDC:oleic acid ratio of 1:0.17:0.57 (g:g:mL). Yield of CHIT-125-L₁₅: 78%.

**¹H NMR** (D₂O, 500 MHz, 25 ºC, TFA (1%)) (selected signals): -CO-CH₂-(CH₂)₅-CH₂-CH=CH-CH₂-(CH₂)₆-CH₃ (0.88 ppm); -CO-CH₂-(CH₂)₅-CH₂-CH=CH-CH₂-(CH₂)₆-CH₃ (1.16-1.67 ppm); -CO-CH₂-(CH₂)₅-CH₂-CH=CH-CH₂-(CH₂)₆-CH₃ (1.97-2.06 and 2.22-2.31); H₃C-CONH- (2.07 ppm); H₂ (3.18 ppm) H₃₋₆ (3.52-4.00 ppm) and -CO-CH₂-(CH₂)₅-CH₂-CH=CH-CH₂-(CH₂)₆-CH₃ (5.32 ppm).

### Step 2: CHIT-125-L₁₅-O-1.6 was prepared from CHIT-125-L₁₅ according to Procedure 2, using a CHIT-125-L₁₅:chlorosulfonic acid ratio of 1:2.8 (g:mL).. Yield of CHIT-125-L₁₅-O-1.6: 89%

**¹H NMR** (D₂O, 500 MHz, 25 ºC) (selected signals): -CO-CH₂-(CH₂)₅-CH₂-CH=CH-CH₂-(CH₂)₆-CH₃ (0.73 ppm); -CO-CH₂-(CH₂)₅-CH₂-CH=CH-CH₂-(CH₂)₆-CH₃ (0.97-1.26 ppm); H₃C-CONH- (1.92 ppm); H₂ (3.24 ppm); H₃(H) and H_{6a-b} (H) (3.45-3.92 ppm); H₃ (SO₃⁻) and H_{6a-b} (SO₃⁻) (3.93-4.51 ppm) and H₁ (4.81 ppm).

**Elemental analysis:** %S= 14.18; %N= 3.89; S/N= 1.6.

### 3.2. Synthesis of CHIT-X-L_{Y}-N-Z polysaccharides3.2.1. CHIT-15-L₁₅-N-0.7 (X= 15; Y= 15; Z= 0.7)

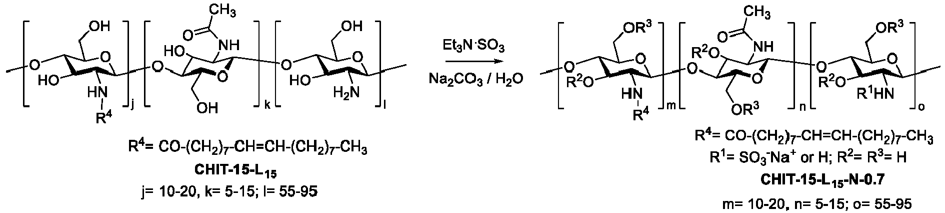

**CHIT-15-L₁₅-N-0.7** was prepared from **CHIT-15-L₁₅** according to Procedure 3, using a CHIT-15-L₁₅: Na₂CO₃:Me₃N·SO₃ ratio of 1:0.3:0. 7 (g:g:g). Yield of **CHIT-15-L₁₅-N-**0.7: 77%

**¹H NMR** (D₂O + Na₂CO₃, pH=9.0, 500 MHz, 70º) (selected signals): -CO-CH₂-(CH₂)₅-CH₂-CH=CH-CH₂-(CH₂)₆-CH₃ (0.75 ppm); -CO-CH₂-(CH₂)₅-CH₂-CH=CH-CH₂-(CH₂)₆-CH₃ (0.91-1.66 ppm); H₃C-CONH- (1.91 ppm); H₂ (H) (2.68 ppm); H₂ (SO₃⁻) (2.95 ppm); H₃-H₆ (3.43-3.92 ppm); H₁ (4.45 ppm) and -CO-CH₂-(CH₂)₅-CH₂-CH=CH-CH₂-(CH₂)₆-CH₃ (5.20 ppm).

**Elemental analysis:** %S= 7.83; %N= 3.84; S/N= 0.7.

### 3.3. Synthesis of CHIT-X-O-Z polysaccharides

### 3.3.1. CHIT-5-O-1.8 (X= 5; Z= 1.8)

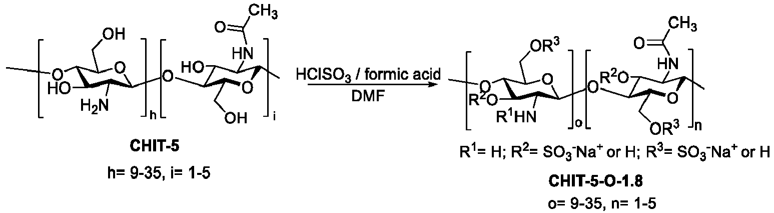

**CHIT-5-O-1.8** was prepared from **CHIT-5** according to Procedure 2, using a CHIT-5:chlorosulfonic acid ratio of 1:2.8 (g:mL). Yield of **CHIT-5-O-1.8:** 65%.

**¹H NMR** (D₂O, 500 MHz) (selected signals): H₃C-CONH- (1.92 ppm); H₂ (3.40 ppm); H_{6(a,b)} (H) and H₃ (H) (3.55-3.83 ppm) and H_{6(a,b)} (SO₃⁻) and H₃ (SO₃⁻) (3.82-4.38 ppm). **Elemental analysis:** S(%)= 12.33; N(%)= 2.94. S/N= 1.8.

### 3.3.2. CHIT-15-O-1.6 (X= 5; Z= 1.6)

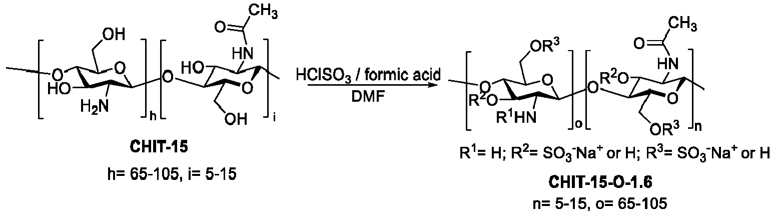

**CHIT-15-O-1.6** was prepared from **CHIT-15** according to Procedure 2, using a CHIT-15:chlorosulfonic acid ratio of 1:2.8 (g:mL). Yield of **CHIT-15-O-1.6:** 95%.

**¹H NMR** (D₂O, 500 MHz) (selected signals): H₃C-CONH- (2.06 ppm); H₂ (3.28 ppm); H_{6(a,b)} (H) and H₃ (H) (3.44-3.96 ppm) and H_{6(a,b)} (SO₃⁻) and H₃ (SO₃⁻) (4.02-4.51 ppm) and H₁ (4.87 ppm).

**Elemental analysis:** S(%)= 13.46; N(%)= 3.55. S/N= 1.6.

### 3.4. Synthesis of CHIT-X-N-Z polysaccharides

### 3.4.1. CHIT-15-N-0.7 (X= 15, Z= 0.7)

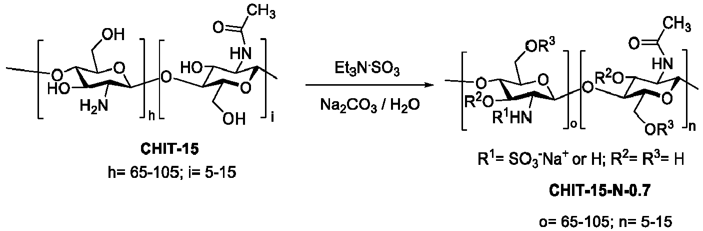

**CHIT-15-N-0.7** was prepared from **CHIT-15** according to Procedure 3, using a CHIT-15: Na₂CO₃:Me₃N·SO₃ ratio of 1:0.3:0. 7 (g:g:g). Yield of **CHIT-15-N-0.7:** 89%.

**¹H NMR** (D₂O + Na₂CO₃, pH=9.0, 500 MHz, 70º) (selected signals): H₃C-CONH-(2.04 ppm); H₂(H) (2.70 ppm); H₂ (SO₃⁻) (3.09 ppm); H₃-H₆ (3.55-4.18 ppm) and H₁ (4.58 ppm).

**Elemental analysis:** S(%)= 8.63; N(%)= 5.2. Z= 0.7.

### 3.5. Synthesis of CHIT-X-N,O-Z polysaccharides

### 3.5.1. CHIT-5-N,O-2.5 (X= 5, Z= 2.5)

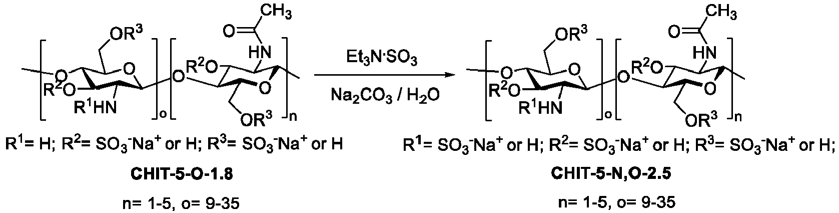

**CHIT-5-N,O-2.5** was prepared from **CHIT-5-O-1.8** according to Procedure 3, using a CHIT-5-O-1.8: Na₂CO₃:Me₃N·SO₃ ratio of 1:0.3:0. 7 (g:g:g).. Yield of **CHIT-5-N,O-2.5:** 61%.

**¹H NMR** (D₂O + Na₂CO₃, pH=9.0, 500 MHz, 70º) (selected signals): H₃C-CONH-(1.92 ppm); H₂(H) (3.16 ppm); H₂(SO₃⁻) (3.42 ppm); H_{6(a,b)} (H) and H₃ (H) (3.49-3.98 ppm) and H_{6(a,b)} (SO₃⁻) and H₃ (SO₃⁻) (3.99-4.53 ppm).

**Elemental analysis:** S(%)= 14.44; N(%)= 2.77. Z= 2.5.

### 3.5.2. CHIT-15-N,O-2.0 (X= 15, Z= 2.0)

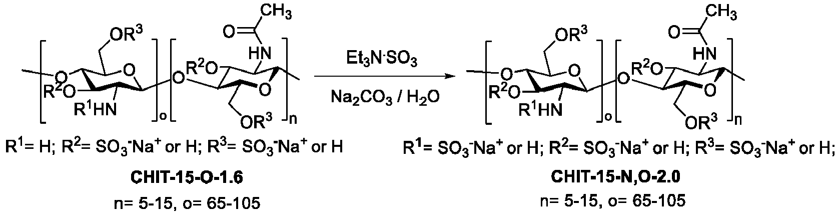

**CHIT-15-N,O-2.0** was prepared from **CHIT-15-O-1.6** according to Procedure 3, using a CHIT-15- O-1.6: Na₂CO₃:Me₃N·SO₃ ratio of 1:0.3:0. 7 (g:g:g).. Yield of **CHIT-15-N,O-2.0:** 65%.

**¹H NMR** (D₂O + Na₂CO₃, pH=9.0, 500 MHz, 70º) (selected signals): H₃C-CONH-(1.92 ppm); H₂(H) (3.02 ppm); H₂ (SO₃⁻) (3.20 ppm); H_{6(a,b)} (H) and H₃ (H) (3.43-3.96 ppm) and H_{6(a,b)} (SO₃⁻) and H₃ (SO₃⁻) (4.04-4.41 ppm).

**Elemental analysis:** S(%)= 14.52; N(%)= 3.09. Z= 2.00.

### 4. BIOLOGICAL SCREENING

**Pseudotyped VSV (SARS-CoV-2-S) Assay Description:** Pseudotyped vesicular stomatitis virus (VSV) carrying a codon-optimized S protein of the Wuhan SARS-CoV-2 strain and encoding both GFP and firefly luciferase was produced and tittered on Vero cells as previously described (Gozalbo-Rovira, R. et al. J Clin Virol. (2020) 131, 104611). For antiviral assays, 1000 focus forming units were either premixed with the compounds at the indicated time for 1 hour at 37 °C before the addition of the mixture to Vero cells previously plated in a 96-well plate (pre-treatment of virus inoculum), added to cells at the same time as compound treatment (concomitant treatment), or added to cells for 1 hour after which the virus was removed and the compounds were added (post infection treatment). In all conditions, a mock treatment with the dilutent was included (DMEM). After 16 hours, toxicity was assessed by adding resazurin (Sigma-Aldrich, R7017) to a final concentration of 0.4 mg/mL, incubation for 1 hour at 37 °C and fluorescence was read on a Tecan Spark microplate reader with an excitation of 535 nm and emission of 595 nm. Finally, 100 µL of Pierce Firefly Luc One-Step Glow Assay Kit (Thermo Scientific, 16197) were added and luminescence read on the Tecan Spark reader. All compounds were tested in triplicate and toxicity or antiviral activity derived by comparison to control treated cells.

**SARS-CoV-2 Assay Description:** A SARS-CoV-2 virus carrying the D614G mutation in the S protein was used. The virus was amplified at a multiplicity of infection of 0.001 for 60 hours on Vero cells and tittered via limiting dilution. For antiviral assays, 10⁴ 50% tissue culture infectious dose (TCid50) of the virus were premixed with the compounds for 1 hour at 37°C. Subsequently, the mixture was used to infect Vero cells in a 24-well plate for 1 hour at 37 °C, after which the inoculum was removed and media containing the compound added. Virus production was assessed following 24 and 48 hours. For this, 50 µL of tissue culture supernatant was used to extract viral RNA using the Zymo quick-RNA Viral kit, and viral RNA quantified via qRT-PCR using the GoTaq probe 1 step RT-qPCR mix (Promega) and the CDC N1 primers. A plasmid encoding the N protein was used to derive the standard curve (Thao, T.T.N. et al. Nature (2020) 582, 561).

**RSV Assay Description:** Compounds were tested for antiviral activity against Respiratory Syncytial Virus (RSV) using a recombinant RSV expressing the fluorescent protein mKate (RSV-A2line19F-mKate) (Moors, M.L. et al. J. Virol. (2009) 83, 4185). Briefly, 1000 focus forming units were premixed with the compounds at the indicated concentrations for 1 hour at 37 °C before the addition of the mixture to Vero or A549-ACE2 cells previously plated in a 96-well plate. A mock treatment with the diluent was included (DMEM). After 24 hours, virus infection was quantified by examining red fluorescence on a live-cell microscope (Incucyte S3, Sartorius). Finally, toxicity was assessed by adding resazurin (Sigma-Aldrich, R7017) to a final concentration of 0.4 mg/mL, incubation for 1 hour at 37 °C and fluorescence was read on a Tecan Spark microplate reader with an excitation of 535 nm and emission of 595 nm. All compounds were tested in triplicate and toxicity or antiviral activity derived by comparison to control-treated cells.

### 4.1. Results of the antiviral screening

• ***Pretreatment of the** pseudotyped **virus (SARS-CoV-2-S) prior to infection.*** The results of the luminescence measurements of the experiment are collected in Table 1 and represented in Figure 1.

**Table 1**

| **Compound** | **Avg-SARS-CoV-2-S-Vero Cells 1 mg/mL** | **Avg-cytotoxicity-Vero cells 1 mg/mL** |
|---|---|---|
| **Heparin** | 0.891193182 | 1.1255996 |
| **k-Carrageenan** | 0.322159091 | 1.1590521 |
| **l- Carrageenan** | 0.325284091 | 1.1974253 |
| **CHIT -5-0-1.8** | 0.45428157 | 0.91252528 |
| **CHIT -5-N,O-2.5** | 0.1808418 | 0.90233925 |
| **CHIT-15-O-1.6** | 0.41212505 | 1.075907 |
| **CHIT-15-N-0.7** | 0.20227273 | 1.05251514 |
| **CHIT-15-N,O-2.0** | 0.49090909 | 1.11305011 |
| **CHIT-15-L₁₅-O-1.6** | 0.02244318 | 1.20066753 |
| **CHIT-15-L₃₀-O-1.7** | 0.01973875 | 1.20206986 |
| **CHIT-15-L₁₅-N-O.7** | 0.01505682 | 0.95635341 |
| **CHIT-20-L₁₅-O-1.7** | 0.4743106 | 1.50917469 |
| **CHIT-30-L₁₅-O-1.7** | 0.42002903 | 1.48374135 |
| **CHIT-50-L₁₅-O-1.6** | 0.05486212 | 1.3736179 |
| **CHIT-90-L₁₅-O-1.7** | 0.34542816 | 1.35366307 |
| **CHIT-125-L₁₅-O-1.6** | 0.07605225 | 1.35134106 |

See figure 1. The results show that the entry rate of SARS-CoV-2 spike pseudotype virus is reduced by 98%-51% compared to control when treated with chitosan sulfate derivatives before infection.
• ***Pretreatment of the virus inoculum prior to infection (Pre_drug_treat), concomitant drug treatment and infection (Concomintant_drug_treat) and post-infection drug treatment (Post_drug_treat).*** The results of the luminescence measurements of the experiment are collected in Table 2 and represented in Figure 2.

**Table 2**

| **Compound** | **Treatment** | **Avg, vs, Control Treatment** |
|---|---|---|
| **CHIT-15-N,O-2.0** | Post_drug_treat | 0.545802534 |
| | Pre_drug_treat | 0.3667045 |
| | Concomintant_drug_treat | 0.625104271 |
| | Avg, Cell Viability | 0.829963925 |
| **CHIT-15-N-0.7** | Post_drug_treat | 0.846356917 |
| | Pre_drug_treat | 0.20227273 |
| | Concomintant_drug_treat | 0.623900885 |
| | Avg, Viability | 0.907732161 |
| **CHIT-15-L₁₅-O-1.6** | Post_drug_treat | 0.901531151 |
| | Pre_drug_treat | 0.013510742 |
| | Concomintant_drug_treat | 0.054918156 |
| | Avg, Viability | 0.976823496 |

See figure 2. The results show that no significant reduction in viral entry is observed when treatment with chitosan sulfate derivatives is concominant (Concomintant_drug_treat) or posinfection (Post_drug_treat), while a significant reduction is observed when treatment with chitosan sulfate derivatives is preinfection (Pre_drug_treat). Taken together, these results are evidence that chitosan sulfate derivatives prevent virus entry, the SARS-CoV-2 process that the SARS-CoV-2 pseudovirus can simulate.
• ***Pretreatment of SARS-CoV-2 prior to infection.*** Results of the viral RNA measurements in qRT-PCR of the experiments are showed in Table 3 and represented in Figure 3.

**Table 3**

| **Compound** | **Avg-SARS-CoV-2** |
|---|---|
| **Growing medium (DMEM)** | 18718800.2 |
| **Heparin** | N.D.^{[1]} |
| **k-carrageenan** | 23465.417 |
| **CHIT-15-O-1.6** | N.D.^{[1]} |
| **CHIT-15-N,O-2.0** | N.D.^{[1]} |
| **CHIT-15-L₁₅-O-1.6** | N.D.^{[1]} |
| **CHIT-15-L₃₀-O-1.7** | N.D.^{[1]} |
| **CHIT-15-L₅₀-O-1.6** | 29267.88 |
| **CHIT-50-L₁₅-O-1.6** | N.D.^{[1]} |
| **CHIT-125-L₁₅-O-1.6** | N.D.^{[1]} |

| | |
|---|---|
| [1] N.D.= not detected | |

See Figure 3. The results show that when Vero cells are treated with chitosan sulfate derivatives prior to infection, viral RNAs are either not detected or are detected at extremely low levels, demonstrating that the derivatives have a preventive effect and protect the cells from infection with SARS-CoV-2.
• ***Pretreatment of RSV prior to infection of Vero cells.*** Results of the final red fluorescence measurements at different product concentrations (1, 0.5 and 0.1 mg/mL) are showed in Table 4 and represented in Figure 4 and cell viability results at the same concentrations are showed in Table 5 and represented in Figure 5.

**Table 4**

| **Compound** | **Avg-RSV-Vero cells** | | |
|---|---|---|---|
| | **1 mg/mL** | **Concentration 0.5 mg/mL** | **0.1 mg/mL** |
| **CHIT-5-N,O-2.5** | 0.00285254 | 0.00122086 | 0.00370173 |
| **CHIT-5-L₁₅-O-1.6** | 0.00957781 | 0.01200549 | 0.02896573 |
| **CHIT-15-N,O-2.0** | 0.00066751 | 0.00060928 | 0.00078871 |
| **CHIT-15-L₁₅-O-1.6** | 0.00137345 | 0.00139302 | 0.00775544 |
| **CHIT-15-L₁₅-N-O.7** | 0.00325274 | 0 | 0.00094336 |
| **CHIT-15-L₃₀-O-1.7** | 0 | 4.18E-05 | 0.00067619 |
| **CHIT-20-L₁₅-O-1.7** | 0.00505058 | 0.00122086 | 0.0032439 |
| **CHIT-30-L₁₅-O-1.7** | 0.00123688 | 0.00060928 | 0.00407047 |
| **CHIT-50-L₁₅-O-1.6** | 0.001457 | 0.00182944 | 0.00330875 |
| **CHIT-125-L₁₅-O-1.6** | 0.00711645 | 0.00376962 | 0.01193988 |

See Figure 4. The results show that when Vero cells are treated with chitosan sulfate derivatives prior to infection, RSV infection is either not detected or is detected at extremely low levels after 24 hours. This indicate that derivatives exert preventive effect that protect cells against infection with RSV,

**Table 5**

| **Compound** | **Avg-cell viability-Vero cells** | | |
|---|---|---|---|
| | **1 mg/mL** | **Concentration 0.5 mg/mL** | **0.1 mg/mL** |
| **CHIT-5-N,O-2.5** | 1.1105908 | 1.01857405 | 0.99552755 |
| **CHIT-5-L₁₅-O-1.6** | 0.97277041 | 0.98136701 | 1.09407877 |
| **CHIT-15-N,O-2.0** | 0.98198296 | 1.01857405 | 0.99552755 |
| **CHIT-15-L₁₅-O-1.6** | 1.17096622 | 1.05653311 | 1.01723452 |
| **CHIT-15-L₁₅-N-O.7** | 1.03774142 | 1.04881292 | 0.94038483 |
| **CHIT-15-L₃₀-O-1.7** | 1.1294523 | 1.05959921 | 1.06294336 |
| **CHIT-20-L₁₅-O-1.7** | 1.24901504 | 1.01537667 | 1.06376796 |
| **CHIT-30-L₁₅-O-1.7** | 1.28253772 | 1.01992948 | 1.05693431 |
| **CHIT-50-L₁₅-O-1.6** | 1.20369948 | 0.98798268 | 1.08156431 |
| **CHIT-125-L₁₅-O-1.6** | 1.13058896 | 1.05653311 | 1.01723452 |

See Figure 5. The results show that, compared with untreated group, there was no significant difference in absorbance of chitosan sulfate derivatives treatment for 24 h at the concentration ranged from 0.1 to 1.0 mg/mL, respectively, indicating that there was no significant cytotoxicity within the concentration range in Vero cells.
• ***Pretreatment of RSV prior to infection of A549-ACE2 cells.*** Results of the Results of the final red fluorescence at different product concentrations (1, 0.5 and 0.1 mg/mL) are showed in Table 6 and represented in Figure 6 and cell viability results at the same concentrations are showed in Table 7 and represented in Figure 7.

**Table 6**

| **Compound** | **Avg-RSV- A549-ACE2 cells infection** | | |
|---|---|---|---|
| | **1 mg/mL** | **Concentration 0.5 mg/mL** | **0.1 mg/mL** |
| **CHIT-5-N,O-2.5** | 0.00131849 | 0.00785431 | 0.00642882 |
| **CHIT-5-L₁₅-O-1.6** | 0.00746974 | 0.02249743 | 0.04620835 |
| **CHIT-15-N,O-2.0** | 0.00109884 | 0.00438443 | 0.00044958 |
| **CHIT-15-L₁₅-O-1.6** | 0.00026915 | 0.01523287 | 0.00676264 |
| **CHIT-15-L₁₅-N-O.7** | 0.00827159 | 0.00798374 | 0.00806372 |
| **CHIT-15-L₃₀-O-1.7** | 2.04E-05 | 0.00252574 | 0.00451761 |
| **CHIT-20-L₁₅-O-1.7** | 0.00294736 | 0.00054971 | 0.00071277 |
| **CHIT-30-L₁₅-O-1.7** | 0.0015572 | 0 | 0.00141032 |
| **CHIT-50-L₁₅-O-1.6** | 0.0007254 | 0.00927569 | 0.003355 |
| **CHIT-125-L₁₅-O-1.6** | 0.00399015 | 0.00636666 | 0.00635827 |

See Figure 6. The results show that when A549-ACE2 cells are treated with chitosan sulfate derivatives prior to infection, RSV infection is either not detected or is detected at extremely low levels at 24 hours, which indicates that derivatives exert preventive effect that protect cells against infection with RSV.

**Table 7**

| **Compound** | **Avg-cell viability- A549-ACE2 cells** | | |
|---|---|---|---|
| | **1 mg/mL** | **Concentration 0.5 mg/mL** | **0.1 mg/mL** |
| **CHIT-5-N,O-2.5** | 1.16945388 | 1.09234168 | 1.19533101 |
| **CHIT-5-L₁₅-O-1.6** | 0.62718295 | 1.19768451 | 1.16330542 |
| **CHIT-15-N,O-2.0** | 0.84214463 | 0.94154265 | 0.99419195 |
| **CHIT-15-L₁₅-O-1.6** | 0.98140255 | 0.88430379 | 0.95539459 |
| **CHIT-15-L₁₅-N-O.7** | 0.82647655 | 0.67987824 | 0.47815491 |
| **CHIT-15-L₃₀-O-1.7** | 1.31802017 | 1.30951278 | 1.24530456 |
| **CHIT-20-L₁₅-O-1.7** | 1.10478828 | 1.2999341 | 1.08291015 |
| **CHIT-30-L₁₅-O-1.7** | 1.17149637 | 1.33418151 | 1.31434389 |
| **CHIT-50-L₁₅-O-1.6** | 1.03529408 | 1.28987828 | 1.27057091 |
| **CHIT-125-L₁₅-O-1.6** | 0.76007364 | 1.07070261 | 0.94562146 |

See Figure 7. The results show that, compared with untreated group, there was no significant difference in absorbance of chitosan sulfate derivatives treatment for 16 h at the concentration ranged from 0.1 to 1.0 mg/mL, respectively, indicating that there was no significant cytotoxicity within the concentration range in A549-ACE2 cells.

Enveloped virus, such as coronaviruses (e.g. SARS-CoV-2 or MERS), human respiratory syncytial virus (RSV), human metapneumovirus, or influenza virus can produce severe respiratory infections by infecting and replicating in the airway epithelia. One of the modalities for their treatment include blocking the initial step of viral infection namely, the binding of the virus to its receptor/s on human cells.

In the context of coronaviruses, it is well-known that the Spike protein (S) recognizes and binds to host cells receptors, and the conformational changes induced in the S protein facilitate the fusion of the viral envelope with the host cell membranes. In this context, it has been reported that these viruses employ HS-proteoglycans as adhesion molecules to increase the density of the virus on cell surface and possibly facilitate the interaction between the S protein, the main mediator of viral entry, and its cellular receptor, ACE2.

Based on these results, in the context of this invention, we have hypothesized that polysaccharides that mimic the structure of heparan sulfates (HS) could bind the virus forming a shielding layer on the virus surface by their interaction with S protein, inhibiting the process of infection. To test this hypothesis, sulfated derivatives of chitosan were synthesized and their antiviral effects in cell culture systems were investigated.

Chitosan sulfates grafted with oleic acid chains have demonstrated to be effective inhibitors of these viral infections, with potential therapeutic value in the treatment of the viral diseases. The high activity of grafted chitosan sulfates (CHIT-X-L_{Y}-O-Z) relative to other polysaccharides tested (CHIT-X-N-Z) may be a result of multivalent interactions between the polysaccharide and viral particle. While CHIT-X-N-Z are linear polysaccharides, CHIT-X-L_{Y}-O-Z are branched, possibly conferring added points of interaction with the viral envelope proteins.

The inhibitory effects of these may be mediated by blocking the interaction of viral proteins with their cellular receptors. To test whether these compounds were antiviral, we examined the ability of pseudotyped virus particles that use the S protein to enter cells to infect in the presence or absence of such compounds. We found that infection was significantly reduced upon addition of the ChS, indicating an antiviral activity. Importantly, addition of some of these compounds following infection resulted in the loss and/or reduction of such antiviral activity, indicating that at least some of these compounds act at the stage of binding and entry. Finally, the antiviral effect of these compounds was validated using SARS-CoV-2 in cell culture (see figure 3). Additionally, it was found that N-sulfated and O-sulfated chitosans grafted with oleic acid chains (CHIT-X-L_{Y}-O-Z and CHIT-X-L_{Y}-N-Z) and N,O-sulfated chitosans (CHIT-X-N,O-Z) prevented RSV infection in Vero cells (see figure 4) and in A549-ACE2 cells (see figure 6), and no evidence of cytotoxicity was observed (see figures 5 and 7). Hence, using two distinct viruses, we demonstrate the general utility of chitosan sulfates as antivirals, highlighting their potential utility as broad-spectrum antivirals against viruses employing HS for entry.

## Claims

1. A compound of formula **I**: 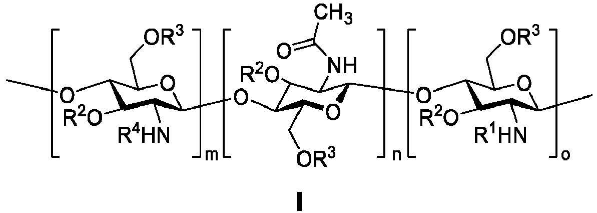 wherein:
each of R¹, R² or R³ independently represents H or -SO₃M; with the proviso that total sulfation degree of compound expressed by Z is of between 0.05 and 3, and wherein
Z is calculated in base to elemental analysis of compound according to this general formula: Z = (S% /32.06)/(N%/14.01); wherein S% and N% represent the percentage of each element by weight
M represents a monovalent cation, preferably pyridinium cation, an ammonium cation or an alkali metal cation;
R⁴ represents -CO-(CH₂)₇-CH=CH-(CH₂)₇-CH₃;
m represents from 0 to 1760 repeating units;
n represents from 1 to 1100 repeating units; and
o represents from 1 to 2100 repeating units.

2. The compound according to claim 1, wherein M is an ammonium cation selected from NH₄⁺, NMe₃H⁺, NEt₃H⁺ and NBu₃H⁺.

3. The compound according to claim 1, wherein M is an alkali metal cation selected from Na⁺, K⁺, Cs⁺ or Li⁺.

4. The compound according to claim 3, wherein M is Na⁺.

5. The compound according to claim 1, selected from:
m= 1-5, n= 1-5, o= 5-40, R¹= H, R²= SO₃⁻Na⁺ or H, R³= SO₃⁻Na⁺ or H, Z= 1.6 (CHIT-5-L₁₅-O-1.6);
m= 0, n= 1-5, o= 9-35, R¹= SO₃⁻Na⁺ or H, R²= SO₃⁻Na⁺ or H, R³= SO₃⁻Na⁺ or H, Z= 2.5 (CHIT-5-N,O-2.5);
m= 0, n= 1-5, o= 9-35, R¹= H, R²= SO₃⁻Na⁺ or H, R³= SO₃⁻Na⁺ or H, Z= 0.5-2.0 (CHIT-5-O-1.8);
m= 10-20, n= 5-15; o= 55-95, R¹= H, R²= SO₃⁻Na⁺ or H, R³= SO₃⁻Na⁺ or H, Z= 1.6 (CHIT-15-L₁₅O-1.6);
m= 20-35, n= 5-15, o= 40-70, R¹= H, R²= SO₃⁻Na⁺ or H, R³= SO₃⁻Na⁺ or H, Z= 1.7 (CHIT-15-L₃₀-O-1.7);
m= 40-70, n= 5-15, o= 20-35, R¹= H, R²= SO₃⁻Na⁺ or H, R³= SO₃⁻Na⁺ or H, Z= 1.6 (CHIT-15-L₅₀-O-1.6);
m= 10-20, n= 5-15; o= 55-95, R¹= SO₃⁻Na⁺ or H, R²= R³= H, Z= 0.7 (CHIT-15- L₁₅-N-0.7);
m= 0, n= 5-15, o= 65-105, R¹= SO₃⁻Na⁺ or H, R²= SO₃⁻Na⁺ or H, R³= SO₃⁻Na⁺ or H, Z= 2.0 (CHIT-15-N,O-2.0);
m= 0, n= 5-15, o= 65-105, R¹= H, R²= SO₃⁻Na⁺ or H, R³= SO₃⁻Na⁺ or H, Z= 1.6 (CHIT-15-O-1.6);
m= 0, n= 5-15, o= 65-105, R¹= SO₃⁻Na⁺ or H, R²= R³= H, Z= 0.7 (CHIT-15-N-0.7);
m= 7-30, n= 5-20, o= 45-125, R¹= H, R²= SO₃⁻Na⁺ or H, R³= SO₃⁻Na⁺ or H, Z= 1.7 (CHIT-20-L₁₅-O-1.7);
m= 15-35, n= 15-40, o= 75-160, R¹= H, R²= SO₃⁻Na⁺ or H, R³= SO₃⁻Na⁺ or H, Z= 1.7 (CHIT-30-L₁₅O-1.7);
m= 33-54, n= 33-54, o= 154-252, R¹= H, R²= R³= SO₃⁻Na⁺ or H, Z= 1.6 (CHIT-50-L₁₅-O-1.6)
m= 69-90, n= 92-120, o= 299-390, R¹= H, R²= SO₃⁻Na⁺ or H, R³= SO₃⁻Na⁺ or H, Z= 1.7 (CHIT-90-L₁₅O-1.7);
m= 105-173, n= 105-173, o= 490-806, R¹= H, R²= SO₃⁻Na⁺ or H, R³= SO₃⁻Na⁺ or H, Z= 1.6 (CHIT-125-L₁₅-O-1.6).

6. The compound according to claim 1, selected from:
m= 10-20, n= 5-15; o= 55-95, R¹= H, R²= SO₃⁻Na⁺ or H, R³= SO₃⁻Na⁺ or H, Z= 1.6 (CHIT-15-L₁₅-O-1.6);
m= 20-35, n= 5-15, o= 40-70, R¹= H, R²= SO₃⁻Na⁺ or H, R³= SO₃⁻Na⁺ or H, Z= 1.6 (CHIT-15-L₃₀-O-1.7);
m= 0, n= 5-15, o= 65-105, R¹= SO₃⁻Na⁺ or H, R²= R³= H, Z= 0.7 (CHIT-15-N-0.7);
m= 0, n= 5-15, o= 65-105, R¹= SO₃⁻Na⁺ or H, R²= SO₃⁻Na⁺ or H, R³= SO₃⁻Na⁺ or H, Z= 2.0 (CHIT-15-N,O-2.0);
m= 0, n= 5-15, o= 65-105, R¹= H, R²= SO₃⁻Na⁺ or H, R³= SO₃⁻Na⁺ or H, Z= 1.6 (CHIT-15-0-1.6);
m= 15-35, n= 15-40, o= 75-160, R¹= H, R²= SO₃⁻Na⁺ or H, R³= SO₃⁻Na⁺ or H, Z= 1.7 (CHIT-30-L₁₅O-1.7); and
m= 33-54, n= 33-54, o= 154-252, R¹= H, R²= SO₃⁻Na⁺ or H, R³= SO₃⁻Na⁺ or H, Z= 1.6 (CHIT-50-L₁₅O-1.6).

7. The compound according to claim 1, selected from:
m= 10-20, n= 5-15, o= 55-95, R¹= H, R²= SO₃⁻Na⁺ or H, R³= SO₃⁻Na⁺ or H, Z= 1.7 (CHIT-L₁₅-O-1.6);
m= 20-35, n= 5-15, o= 40-70, R¹= H, R²= SO₃⁻Na⁺ or H, R³= SO₃⁻Na⁺ or H, Z= 1.6 (CHIT-15-L₃₀-O-1.7);
m= 15-35, n= 15-40, o= 75-160, R¹= H, R²= SO₃⁻Na⁺ or H, R³= SO₃⁻Na⁺ or H, Z= 1.7 (CHIT-30-L₁₅O-1.7); and
m= 33-54, n= 33-54, o= 154-252, R¹= H, R²= SO₃⁻Na⁺ or H, R³= SO₃⁻Na⁺ or H, Z= 1.6 (CHIT-50-L₁₅O-1.6).

8. A pharmaceutical composition comprising a compound of formula I according to any of claims 1 to 7 or a pharmaceutically acceptable salt thereof, together with a pharmaceutically acceptable vehicle, and optionally another active substance.

9. A compound of formula I according to any of claims 1 to 7 or a pharmaceutically acceptable salt thereof for use in therapy.

10. A compound of formula **I** according to any of claims 1 to 7 or a pharmaceutically acceptable salt thereof for use in the treatment and/or prevention of a viral infections.

11. The compound for use according to claim 10 or a pharmaceutically acceptable salt thereof, wherein the viral infection is caused by SARS-CoV-2, respiratory syncytial virus (RSV), SARS-CoV-1, MERS, human Metapneumovirus (hMPV), Ebola Virus (EBOV), Herpes Simplex Virus (HSV), Human papillomavirus (HPV) and Dengue Virus (DENV).

12. The compound for use according to claim 11 or a pharmaceutically acceptable salt thereof, wherein the viral infection is caused by SARS-CoV-2 or respiratory syncytial virus (RSV).
